# NEW EUROPEAN PATENT SPECIFICATION

(11) **EP 2 558 525 B2**
(45) Date of publication and mention of the opposition decision: **04.04.2018**
(45) Mention of the grant of the patent: 17.12.2014
(21) Application number: 11718183.4
(22) Date of filing: 14.04.2011
(51) Int. Cl.: C08K 5/17, A61K 8/44, C08L 33/06, C08L 35/02

(54) **THICKENED AMINO ACID SURFACTANT COMPOSITIONS AND METHODS THEREFOR**
VERDICKTE OBERFLÄCHENAKTIVE AMINOSÄURENZUSAMMENSETZUNGEN UND VERFAHREN FÜR DIESE
COMPOSITIONS TENSIO-ACTIFS D' AMINOACIDES ÉPAISSIES ET MÉTHODES POUR CELLES-CI

(30) Priority: 14.04.2010 US 324248 P
(43) Date of publication of application: 20.02.2013
(73) Proprietor: Lubrizol Advanced Materials, Inc., Cleveland, OH 44141-3247 (US)
(72) Inventor: YAO, Ge, Broadview Heights Ohio 44147 (US); LIU, Xin, 200093, Shanghai (CN); HE, Xiaoqun, Shanghai 201108 (CN); TJO, Kwe Sung, Singapore 467464 (SG)
(74) Representative: dompatent von Kreisler Selting Werner - Partnerschaft von Patent- und Rechtsanwälten mbB
(86) International application number: PCT/US2011/032390
(87) International publication number: WO 2011/130460

(56) References cited:
- EP-A1- 1 510 204
- EP-A1- 1 559 774
- EP-A1- 1 917 954
- EP-A1- 2 039 346
- EP-A2- 1 010 422
- WO-A1-2008/138393

## Description

### FIELD OF THE INVENTION

The present invention relates to surfactant-polymer blends that are useful in a variety of personal care, health care, home care and cleansing products. The surfactant-polymer blends of the present invention are useful in a variety of products including, but not limited to, personal care products, health care products, household care products, institutional and industrial care products, and industrial applications.

### BACKGROUND OF THE INVENTION

Surfactants are widely used in aqueous based personal care, household care and industrial and institutional care formulations as wetting agents, detergents, and emulsifiers. In personal care cleansing products (e.g., shampoos, body washes, facial cleansers, liquid hand soaps, etc.), household care cleaning products (e.g., hard surface cleaners, laundry detergents, dish soaps, automatic dish washer detergents, shower cleansers, bathroom cleansers, car wash detergents, etc.) and industrial and institutional care cleaners (high strength cleaners, detergents, etc.) the surfactant package is one of the most important components in the detersive formulation. These compositions generally comprise a mixture of one or more surfactants as the active detersive ingredient. The surfactant: 1) improves the wetability of the soiled substrate; 2) loosens soil and/or sebum from the substrate; and 3) emulsifies, solubilizes and/or suspends the loosened soil/sebum particles in the aqueous wash medium.

Although in principle any surfactant class (e.g., cationic, anionic, nonionic, amphoteric) is suitable in cleansing or cleaning applications, in practice most personal care cleansers and household cleaning products are formulated with anionic surfactants or with a combination of an anionic surfactant as the primary detersive agent with one or more secondary surfactants selected from the other surfactant classes. Anionic surfactants are often used as detersive agents in cleansers and cleaning products because of their excellent cleaning and foaming properties. From the consumer's perspective, the amount and stability of the foam is directly related to the perceived cleaning efficiency of the composition. The larger the foam volume produced and the more stable the foam, the more efficient the apparent cleaning action of the composition is in the mind of the consumer. Anionic surfactants traditionally utilized in these formulations include alkyl sulfates and alkyl benzene sulfonates. While these surfactants are efficient detersive agents, produce large foam volumes and possess excellent foam stability properties, they are severe ocular irritants and are capable of causing mild to moderate dermal irritation to some sensitized persons. Accordingly, it has become more and more important to consumers that aqueous cleansing compositions are high foaming as well as mild.

It is known that the irritation caused by anionic sulfates can be reduced by ethoxylation. However, the reduction in irritation is accompanied by a corresponding reduction in foam volume. For example, sodium lauryl sulfate, a high foaming surfactant, causes significant eye irritation. In contrast, sodium laureth-12 sulfate (the corresponding ethoxylate containing 12 ethoxy groups) is almost completely non-irritating, but is a poor foaming agent (see Schoenberg, "Baby Shampoo," Household & Personal Products Industry 60 (September 1979)). The poor foaming properties of ethoxylated alkyl sulfates are reported in many other publications. For example, U.S. Patent No. 4,132,678 discloses that the foaming properties of alkyl (C₁₀ to C₁₈) sulfates are drastically reduced if more than 5 ethoxy groups are added to the molecule. Additional attempts to attenuate the adverse irritant effects of anionic surfactants have been made by replacing some of the foam generating anionic surfactant with very mild secondary surfactants. The anionic surfactant is utilized in conjunction with a nonionic and/or an amphoteric surfactant as disclosed in U.S. Patent No. 4,726,915. However, reducing the amount of anionic surfactant in a cleansing or cleaning composition adversely affects the detersive and foaming properties of the composition.

As a result, mild surfactants, such as amino acid surfactants, are being used more frequently to meet consumer requirements for a mild, high foaming cleanser. Amino acid based surfactants are known for their mildness towards the skin and eyes, conditioning benefits to hair and skin, foam generating properties, and the ability to maintain a stable foam profile in the presence of sebum. Moreover, these surfactants are environmentally friendly in that they are derived from naturally occurring renewable resources such as vegetable oils and amino acids. However, compared to ethoxylated alkyl sulfate surfactants, amino acid based surfactants are not easy to formulate because they are hard to thicken and suffer a reduction in foam generating ability when formulated with the commonly available rheology modifiers or thickeners on the market.

A liquid cleanser or cleaner should be formulated to achieve an ideal viscosity while maintaining the ability to generate a good foam profile. The ideal viscosity allows for the controlled handling and dispensing of the product during use as compared to a thinner product. In personal care cleansing applications, a thick, rich, high foaming shampoo or body cleanser is appealing to consumers from a sensory and use perspective. An ideal viscosity profile permits the formulation of an easy to use product that does not run-off when applied to non-horizontal body surfaces. The shear thinning profile of the liquid body cleansing composition should exhibit a higher viscosity at low shear conditions and lower viscosity at high shear conditions to aid in the application and removal of the product from the body. These properties are especially useful if the cleansing compositions are to be topically applied to human skin and hair.

Many rheology modifiers, both natural and synthetic, are known. Natural rheology modifiers include, for example, casein, alginates, gum tragacanth, and modified cellulose, including methyl cellulose, hydroxyethyl cellulose, hydroxypropyl cellulose and carbomethoxy cellulose. These natural products vary in their thickening efficiency and generally provide poor flow and leveling properties; they are also subject to microbial attack and require the additional presence of antimicrobial agents. Synthetic rheology modifiers include various acrylic polymers and maleic anhydride copolymers. Some of these are found to be pH dependent, others are hydrolytically unstable, some require large amounts of thickener to effectively increase viscosity, others are sensitive to various components normally found in aqueous systems, and still others require large amounts of salt to increase the viscosity to a desired or suitable level.

Given the above, there is a need in the art for a rheology modifier suitable for thickening cleansing compositions containing an amino acid based surfactant without adversely affecting the foam profile of the composition. Also, there is an additional need in the art for a thickening system that provides superior clarity, superior flow properties and flow aesthetics at low use levels.

EP 1510204 discloses a cleansing composition including a) from 0.1 percent to 12.5 percent of an anionic surfactant; and b) from 0.1 percent to 8 percent of a hydrophobically modified, crosslinked, anionic acrylic copolymer, wherein the weight ratio of component a) to component b) is 1:1 to 40:1, and the composition is substantially free of amphoteric surfactants. The composition is useful as shampoos, washes, baths, gels, lotions, creams, and the like.

WO 2008/138393 discloses a gel, for cosmetic use, composed of a mixture of a polymer which forms a gel, a surfactant, and propionyl L-carnitine glycinate hydrochloride, useful for treating disturbances of the skin such as cellulite and wrinkles.

EP 2039346 discloses a cosmetic composition that comprises, in a cosmetically acceptable medium:(i) at least one cationic polymer produced by polymerizing a mixture of monomers comprising: a) at least one vinyl monomer (I) substituted with at least one amino group, b) at least one hydrophobic nonionic vinyl monomer, c) at least one associative vinyl monomer, and d) at least one hydroxylated nonionic vinyl monomer; and (ii) at least one starch. It further discloses a cosmetic treatment process for keratinic materials, involving applying, to the materials, the cosmetic composition and then optionally rinsing with water.

EP 1917954 discloses aqueous o/w emulsions having the ability to invert into w/o emulsions by introduction of mechanical energy, comprising (a) oil components, (b) w/o emulsifiers, (c) anionic surfactants, (d) actives, and (e) polymeric stabilizers. The emulsions are useful for making cosmetic or pharmaceutical compositions, particularly sprayable sun care emulsions.

### SUMMARY OF THE INVENTION

The present invention relates the surfactant-polymer blends as defined in the claims.

The surfactant-polymer blends of the present invention are useful in a variety of cleansing and cleaning products including, but not limited to, personal care products, health care products, household care products, institutional and industrial care products, and industrial applications.

The surfactant-polymer blends of the present invention can be utilized to replace anionic surfactants (e.g., alkyl sulfates and alkyl benzene sulfonates, as well as ethoxylated derivatives thereof) in personal care, household care, health care, and institutional and industrial care compositions with their inherent adverse properties, or can be utilized in combination with reduced amounts of anionic surfactants to mitigate these adverse effects while maintaining the desired detersive, foaming, and viscosity properties in such compositions.

The disclosure further relates to a method for stabilizing foam volume in an amino acid surfactant composition comprising adding a sufficient amount of a hydrophobically-modified polymer polymerized from a mixture comprising:
(i) at least one α,β-ethylenically unsaturated carboxylic acid monomer selected from acrylic acid, methacrylic acid, and mixtures thereof;
(ii) at least one nonionic, co-polymerizable α,β-ethylenically unsaturated monomer selected from C₁ to C₄ alkyl acrylate, C₁ to C₄ alkyl methacrylate, and mixtures thereof; and
(iii) at least one ethylenically unsaturated hydrophobically-modified associative monomer selected from cetyl polyethoxylated (meth)acrylate, cetearyl polyethoxylated (meth)acrylate, stearyl polyethoxylated (meth)acrylate, arachidyl polyethoxylated (meth)acrylate, behenyl polyethoxylated methacrylate, cerotyl polyethoxylated (meth)acrylate, montanyl polyethoxylated (meth)acrylate, melissyl polyethoxylated (meth)acrylate, lacceryl polyethoxylated (meth)acrylate, nonylphenol polyethoxylated (meth)acrylate, tristyrylphenol polyethoxylated (meth)acrylate, palmyl polyethoxylated itaconate, stearyl polyethoxylated itaconate, behenyl polyethoxylated itaconate, palmyl polyethoxylated maleate, stearyl polyethoxylated maleate, behenyl polyethoxylated maleate, palmyl polyethoxylated allyl ether, stearyl polyethoxylated allyl ether, behenyl polyethoxylated allyl ether, wherein the polyethoxylated portion of the foregoing monomers contain from 15 to 60 ethylene oxide repeating units. Preferred embodiments of the description are apparent from the dependent claims.

### BRIEF DESCRIPTION OF THE DRAWINGS

Figure 1 is a graph of viscosity curves comparing selected amino acid surfactant-polymer blend formulations.
Figure 2 is a graph of a viscosity curves for selected amino acid surfactant-polymer blend formulations illustrating the deleterious effects of electrolyte addition.
Figure 3 is a graph of viscosity curves comparing selected amino acid surfactant-polymer blend formulations in combination with anionic and amphoteric surtactants.
Figure 4 is a viscosity curve illustrating the synergy effects on selected amino acid surfactant-polymer blend formulations of the invention in combination with an esterified alkoxylated methyl glucoside.
Figure 5 is foam volume plot comparing the surfactant-polymer blend formulations to a formulation blank.
Figure 6 is a foam stability plot comparing the decay of foam volume over time of the surfactant-polymer blend formulations to a formulation blank.

### DETAILED DESCRIPTION OF THE INVENTION

In one embodiment, the surfactant-polymer blend of the present invention is a linear, non-crosslinked, hydrophobically-modified polymethacrylic acid polyelectrolyte polymer. In one embodiment, the surfactant-polymer blends of the present invention possess superior thickening efficiency in a cleansing composition requiring a mild, high foaming detersive agent(s).

As used herein, "moderate amounts of electrolytes" means that the amount of electrolyte, or electrolytes, present in a composition is less than 4 weight percent, less than 3 weight percent, less than 2 weight percent, less than 1.5 weight percent, or even less than 1 weight percent, based on the total amount of the composition.

### Amino Acid Surtactant - Component (a)

In one embodiment of the invention, the amino acid based surfactants useful as mild, high foaming detersive agent(s) are derived from carboxylate salts of α-amino acids wherein the amine group situated on the α-carbon of an amino acid salt is acylated with a C₁₀ to C₂₂ fatty acid derivative. Amino acid based surfactants used in the practice of the present invention include the following surfactants:
the mono- and di- carboxylate salts (e.g., sodium, potassium, ammonium and TEA) of N-acylated glutamic acid, for example, sodium cocoyl glutamate, sodium lauroyl glutamate, sodium myristoyl glutamate, sodium palmitoyl glutamate, sodium stearoyl glutamate, disodium cocoyl glutamate, disodium stearoyl glutamate, potassium cocoyl glutamate, potassium lauroyl glutamate, and potassium myristoyl glutamate; the carboxylate salts (e.g., sodium, potassium, ammonium and TEA) of N-acylated alanine, for example, sodium cocoyl alaninate, and TEA lauroyl alaninate; the carboxylate salts (e.g., sodium, potassium, ammonium and TEA) of N-acylated glycine, for example, sodium cocoyl glycinate, and potassium cocoyl glycinate; the carboxylate salts (e.g., sodium, potassium, ammonium and TEA) of N-acylated sarcosine, for example, sodium lauroyl sarcosinate, sodium cocoyl sarcosinate, sodium myristoyl sarcosinate, sodium oleoyl sarcosinate, and ammonium lauroyl sarcosinate; and mixtures of the foregoing surfactants.

Suitable acyl halides for acylation of the amino acid carboxylate salt include acyl chlorides, bromides, fluorides, and iodides. The acyl halides can be prepared by reacting a saturated or unsaturated, linear or branched C₁₀ to C₂₂ fatty acid with a thionyl halide (bromide, chloride, fluoride, iodide). Representative acyl halides include but are not limited to the acyl chlorides selected from decanoyl chloride, dodecanoyl chloride (lauroyl chloride), cocoyl chloride (coconut oil derived fatty acid chlorides) tetradecanoyl chloride (myristoyl chloride), hexadecanoyl chloride (palmitoyl chloride), octadecanoyl chloride (stearoyl chloride), 9-octadecenoyl chloride (oleoyl chloride), eicosanoyl chloride (arachidoyl chloride), docosanoyl chloride (behenoyl chloride), and mixtures thereof. Other acyl halides include the bromides, fluorides and iodides of the foregoing fatty acids. A method for preparing acyl halides as well as an alternative method for acylating amino acids therewith is set forth in US Patent Application Publication No. 2008/0200704, published on August 21, 2008.

The amount of amino acid based surfactant that can be included in the personal care, household care, and industrial and institutional care compositions herein can range from 3 weight percent to 50 weight percent in one aspect, from 5 weight percent to 40 weight percent in another aspect, from 8 weight percent to 30 weight percent in a further aspect, and from 10 weight percent to 25 weight percent in still a further aspect of the invention, based on the total weight of the composition in which the surfactant is included. All weights are based on 100 percent of active material.

### Hydrophobically-Modified Thickening Polymer - Component (b)

In one embodiment of the invention, the hydrophobically-modified thickening polymer useful in combination with the amino acid based surfactants described above is a hydrophobically-modified alkali-soluble copolymer that contains hydrophobic substituents in the polymer backbone. In one aspect, the polymer component (b) of the present invention is an aqueous emulsion copolymer polymerized from (i) an α,β-ethylenically unsaturated carboxylic acid monomer; (ii) a nonionic, co-polymerizable α,β-ethylenically unsaturated monomer; and (iii) an ethylenically unsaturated hydrophobically-modified associative monomer.

Component (i) is selected from at least one α,β-ethylenically unsaturated carboxylic acid monomer according to Formula (III): 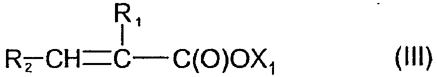 where R₁ is a hydrogen, -CH₂C(O)OX₂, or a linear or branched C₁ to C₁₂ alkyl group; R₂ is a hydrogen, -C(O)OX₂, or a linear or branched C₁ to C₁₂ alkyl group; and where X₁ and X₂ are independently selected from hydrogen, or a linear or branched C₁ to C₁₂ alkyl group, with the proviso that when both X₁ and X₂ are present at least one of X₁ and X₂ is a hydrogen and that when only X₁ is present it is a hydrogen. In another embodiment, R₁ is a hydrogen, -CH₂C(O)OX₂, or a linear or branched C₁ to C₁₀ alkyl group; R₂ is a hydrogen, -C(O)OX₂, or a linear or branched C₂ to C₁₀ alkyl group; and X₁ is hydrogen, or linear or branched C₂ to C₁₀ alkyl group, with the proviso that when both X₁ and X₂ are present at least one of X₁ and X₂ is a hydrogen and that when only X₁ is present it is a hydrogen. In still yet another embodiment of the present invention, R₁ is a hydrogen, -CH₂C(O)OX₂, or a linear or branched C₃ to C₈ alkyl group; R₂ is a hydrogen, -C(O)OX₂, or a linear or branched C₃ to C₈ alkyl group, or; and X₂ is hydrogen, or linear or branched C₃ to C₈ alkyl group, with the proviso that when both X₁ and X₂ are present at least one of X₁ and X₂ is a hydrogen and that when only X₁ is present it is a hydrogen. In still yet another embodiment, R₁ is a hydrogen, methyl, a linear or branched C₁ to C₄ alkyl group, or -CH₂C(O)OX₂; R₂ is a hydrogen, a linear or branched C₁ to C₄ alkyl group, or -C(O)OX₂; and X₁ is hydrogen, or linear or branched C₁ to C₄ alkyl group, with the proviso that when both X₁ and X₂ are present at least one of X₁ and X₂ is a hydrogen and that when only X₁ is present it is a hydrogen.

Examples of component (i) acid monomers include, but are not limited to, acrylic, methacrylic, crotonic, acyloxypropionic, maleic, fumaric, itaconic, aconitic, half- or mono-esters of dibasic or tribasic acids such as the monobutyl ester of maleic acid, or suitable mixtures of two or more thereof. In another embodiment, acrylic acid, methacrylic acid, or a mixture thereof is used as the acid monomer of component (i).

Component (ii) is selected from at least one nonionic, co-polymerizable α,β-ethylenically unsaturated monomer according to Formula (IV): 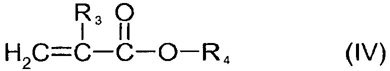 where R₃ is hydrogen, or linear or branched C₁ to C₈ alkyl group; and R₄ is a linear or branched C₁ to C₁₂ alkyl group. In another embodiment, R₃ is hydrogen, or linear or branched C₁ to C₄ alkyl group; and R₄ is a linear or branched C₂ to C₈ alkyl group. In still another embodiment, R₃ is hydrogen or a methyl group; and R₄ is a linear or branched C₁ to C₄ alkyl group. Here, as well as elsewhere in the specification and claims, individual numerical values (including carbon atom numerical values), or limits, can be combined to form additional non-disclosed and/or non-stated ranges.

Examples of component (ii) nonionic monomers include, but are not limited to, C₁ to C₄ alkyl acrylates and methacrylates such as methyl acrylate, ethyl acrylate, butyl acrylate, ethyl methacrylate, or suitable mixtures of two or more thereof.

Component (iii) is selected from at least one ethylenically unsaturated hydrophobically-modified associative monomer according to Formula (V): 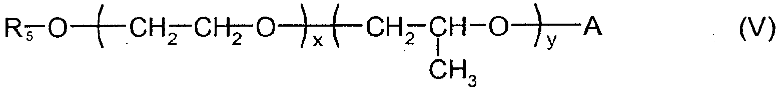 where R₅ is a linear or branched C₃ to C₆₀ alkyl group, or a C₅ to C₃₅ alkaryl group, where the alkaryl group has from 1 to 25 carbon atoms in a linear or branched alkyl moiety and from 4 to 10 carbon atoms in the aryl moiety; x is an integer in the range of 3 to 200; y is an integer in the range of 0 to 100; and A is the residue conforming to Formula (VI): 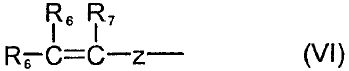 where each R₆ is independently selected from hydrogen, a linear or branched C₁ to C₈ alkyl group, or -C(O)OH; and R₇ is hydrogen, or a linear or branched C₁ to C₈ alkyl group; and Z, the linking group that bonds to Formula (V) above, is -CH₂-, or -C(O)-. In another embodiment, R₅ is a linear or branched C₆ to C₅₅ alkyl group, or a C₇ to C₃₀ alkaryl group, where the alkaryl group has from 2 to 22 carbon atoms in a linear or branched alkyl moiety and from 5 to 8 carbon atoms in the aryl moiety; x is an integer in the range of 6 to 100; y is an integer in the range of 1 to 75; each R₆, is independently selected from hydrogen, a linear or branched C₂ to C₆ alkyl group, or -C(O)OH; and R₇ is hydrogen, a linear or branched C₁ to C₆ alkyl group, or-C(O)OH. In still another embodiment, R₅ is a linear or branched C₈ to C₅ alkyl group, or a C₈ to C₂₂ alkaryl group, where the alkaryl group has from 2 to 14 carbon atoms in a linear or branched alkyl moiety and from 6 to 8 carbon atoms in the aryl moiety; x is an integer in the range of 7 to 75; y is an integer in the range of 2 to about 50; each R₆ is independently selected from hydrogen, a linear or branched C₁ to C₅ alkyl group, or -C(O)OH; and R₇ is hydrogen, a linear or branched C₁ to C₅ alkyl group, or -C(O)OH.

In still yet another embodiment, R₅ is a linear or branched C₁₀ to C₄₀ alkyl group, or a C₈ to C₂₂ alkaryl group, where the alkaryl group has from 2 to 14 carbon atoms in a linear or branched alkyl moiety and from 6 to 8 carbon atoms in the aryl moiety; x is an integer in the range of 10 to 60; y is an integer in the range of 0 to 30; and A is a residue of allyl alcohol or a residue of an acrylic, methacrylic or itaconic acid group. In still yet another embodiment, R₅ is a linear or branched C₁₀ to C₄₀ alkyl group, or a C₈ to C₂₂ alkaryl group, where the alkaryl group has from 2 to 14 carbon atoms in a linear or branched alkyl moiety and from 6 to 8 carbon atoms in the aryl moiety; x is an integer in the range of 16 to 25; y is 0 to 30; and A is a residue of allyl alcohol or a residue of an acrylic, methacrylic or itaconic acid group.

In light of the above, suitable examples of component (iii) (the ethylenically unsaturated hydrophobically-modified associative monomer) that are utilized to form component (c) of the present invention include, but are not limited to, cetyl polyethoxylated (meth)acrylate (CEM), cetearyl polyethoxylated (meth)acrylate (CSEM), stearyl polyethoxylated (meth)acrylate, arachidyl polyethoxylated (meth)acrylate, behenyl polyethoxylated methacrylate (BEM), cerotyl polyethoxylated (meth)acrylate, montanyl polyethoxylated (meth)acrylate, melissyl polyethoxylated (meth)acrylate, lacceryl polyethoxylated (meth)acrylate, nonylphenol polyethoxylated (meth)acrylate (NEM), tristyrylphenol polyethoxylated (meth)acrylate (TEM), palmyl polyethoxylated itaconate (PEI), stearyl polyethoxylated itaconate (SEI), behenyl polyethoxylated itaconate (BEI), palmyl polyethoxylated maleate (PEML), stearyl polyethoxylated maleate (SEM), behenyl polyethoxylated maleate (BEML), palmyl polyethoxylated allyl ether (PEAE), stearyl polyethoxylated allyl ether (SEAE), behenyl polyethoxylated allyl ether (BEAE), where the polyethoxylated portion of the foregoing monomers contain from about 3 to about 200 ethylene oxide repeating units, from about 5 to about 100 ethylene oxide repeating units, or even about 15 to about 60 ethylene oxide repeating units.

According to the invention the amounts of components (i), (ii) and (iii) that are utilized in a suitable polymerization reaction to produce the at least one hydrophobically-modified polymer of component (b) of the blends of the present invention are as follows: (i) from 2.5 weight percent to 75 weight percent of one or more α,β-ethylenically unsaturated carboxylic acid monomers; (ii) from 0 weight percent to 90 weight percent of one or more nonionic, co-polymerizable α,β-ethylenically unsaturated monomers; and (iii) from 0.5 weight percent to 40 weight percent of one or more ethylenically unsaturated hydrophobically-modified associative monomers. In another embodiment, the amounts of components (i), (ii) and (iii) are as follows: (i) from 5 weight percent to 70 weight percent; (ii) from 5 weight percent to 85 weight percent; and (iii) from 1 weight percent to 30 weight percent. In still another embodiment, the amounts of components (i), (ii) and (iii) are as follows: (i) from 10 weight percent to 60 weight percent; (ii) from 10 weight percent to 80 weight percent; and (iii) from 2.5 weight percent to 25 weight percent. In still yet another embodiment, the amounts of components (i), (ii) and (iii) are as follows: (i) from 20 weight percent to 55 weight percent; (ii) from 20 weight percent to 75 weight percent; and (iii) from 5 weight percent to 20 weight percent. In still yet another embodiment, the amounts of components (i), (ii) and (iii) are as follows: (i) from 30 weight percent to 50 weight percent; (ii) from 30 weight percent to 70 weight percent; and (iii) from 7.5 weight percent to 15 weight percent. Here, as well as elsewhere in the specification and claims, individual numerical values (including carbon atom numerical values), or limits, can be combined to form additional non-disclosed and/or non-stated ranges. Although the total amount of each monomer or component of a various "charge" may individually total more then 100 weight percent when each component is taken individually and totaled using the broadest amounts disclosed herein, one of skill in the art will realize that this is not the case.

In another embodiment, components (i), (ii) and (iii) are selected so that they yield polymer component (b) as represented by the formula immediately below. In the instance below, y in Formula (V) is zero. The structure below is for illustrative purposes and is not to be construed as limiting the scope of the invention. The present invention should be broadly construed in accordance with the Formulas and disclosure discussed above. 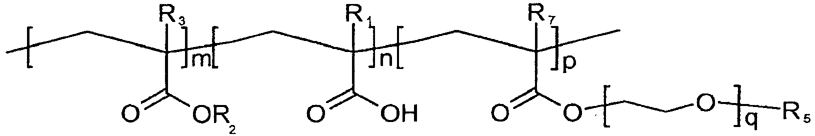 In the foregoing structure R₁, R₂, R₃, R₅ and R₇ are as defined above; m is an integer in the range of 1 to 98; n is an integer in the range of 1 to 98; p is in integer in the range of 1 to 45; and q is an integer in the range of 0 to 100. In another embodiment, R₁, R₂. R₃, R₅ and R₇ are as defined above; m is an integer in the range of 5 to 75; n is an integer in the range of 5 to 75; p is in integer in the range of 2 to 30; and q is an integer in the range of 5 to 75, with the proviso that m + n + p are chosen so that they total 100. In still another embodiment, R₁, R₂. R₃, R₅ and R₇ are as defined above; m is an integer in the range of 10 to 60; n is an integer in the range of 10 to 60; p is in integer in the range of 3 to 20; and q is an integer in the range of from 7 to 60 in one aspect, from 15 to 40 in another aspect, and from 20 to 35 in a further aspect, with the proviso that m + n + p are chosen so that they total 100. Here, as well as elsewhere in the specification and claims, individual numerical values (including carbon atom numerical values), or limits, can be combined to form additional non-disclosed and/or non-stated ranges.

In still yet another embodiment, R₁, R₂. R₃, R₅ and R₇ are as defined above; m is an integer in the range of 1 to 60; n is an integer in the range of 1 to 40; p is in integer in the range of 1 to 20; and q is an integer in the range of 0 to 25, with the proviso that m + n + p are chosen so that they total 100. In still yet another embodiment, when q is zero, R₅ is a linear or branched C₈ to C₄₀ alkyl group. Here, as well as elsewhere in the specification and claims, individual numerical values (including carbon atom numerical values), or limits, can be combined to form additional non-disclosed and/or non-stated ranges.

In another embodiment, polymer component (b) of the blends of the present invention can be crosslinked or non-crosslinked (i.e., linear). In another embodiment, polymer component (b) of the blends of the present invention is non-crosslinked (i.e., linear). In one embodiment, component (b) of the blends of the present invention can be prepared by conventional emulsion polymerization techniques using appropriate emulsifiers for emulsifying the monomers and maintaining the polymer obtained in a stable-dispersed condition. In this instance, polymer component (b) comprises an aqueous emulsion comprising from 1 weight percent to 60 weight percent of one or more of the above polymer components with the remainder of the emulsion being primarily water.

One suitable preparation technique is described in United States Patent No. 4,616,074. In the process disclosed in United States Patent No. 4,616,074, the polymerization is carried out in continuous, semi-continuous or batch fashion with the polymerization reaction initiated at 40°C to 90°C. A thermal decomposition initiator such as ammonium persulfate or potassium persulfate is used, or at lower temperatures a redox initiator such as t-butyl hydroperoxide/bisulfite is used. An emulsifier is normally included in the reaction medium at a concentration of 1 weight percent to 3 weight percent, based on the total weight of the monomer charge, so as to maintain a stable aqueous dispersion. Suitable emulsifiers include, but are not limited to, sodium lauryl sulfate, sodium dodecylbenzene sulfonate, as well as other ammonium and alkali metal alkyl aryl sulfonates and alkyl sulfates. The polymerization is carried out at a pH of less than 5.0 to maintain the insolubility of the copolymer in the continuous water phase. The polymer dispersions have relatively low viscosity even at solids content of from 20 weight percent to 40 weight percent or higher.

Upon addition of an alkali to neutralize at least a portion of the free carboxyl groups, aqueous systems containing one or more polymer components (b) of the present invention markedly thicken. Any alkali can be used to neutralize the copolymers and can be an alkali metal hydroxide such as sodium or potassium hydroxide, sodium carbonate, or other bases such as ammonium hydroxide, methylamine, diethylamine, triethylamine, aminomethyl propanol (AMP) and triethanolamine (TEA). In one embodiment, an effective neutralizing amount of alkali is used and depending on the particular application, it is an amount sufficient to adjust the pH of the mixture to above 6.0. In another embodiment, the amount of neutralizing agent used is an amount sufficient to adjust the pH of the mixture from 6.5 to 14, or from 7 to 12, or from 7.5 to 10, or even from 7.0 to 7.5.

In one embodiment of the invention, the hydrophobically-modified thickening polymer useful in combination with the amino acid based surfactant is commercially available under the INCI designation Acrylates/Beheneth-25 Methacrylate Copolymer. Such polymers are commercially available under the Novethix™ L-10 trade name marketed by Lubrizol Advanced Materials, Inc., Cleveland, Ohio.

The amount of hydrophobically-modified thickening polymer that can be included in the personal care, household care, and industrial and institutional care compositions herein can range from 0.1 weight percent to 10 weight percent in one aspect, from 0.5 weight percent to 6 weight percent in another aspect, and from 0.75 weight percent to 4.5 weight percent in a further aspect, and from 1.2 weight percent to 3 weight percent in still a further aspect of the invention, based on the total weight of the composition in which the thickener is included. All weights are based on 100 percent of active material.

In one embodiment, the surfactant-polymer blends of the invention can be utilized in weight ratios of the at least one amino acid based surfactant, component (a), to the at least one hydrophobically-modified thickening polymer, component (b), that range from 0.3:1 to 500:1 in one aspect, from 1.75:1 to 150:1 in another aspect, from 3.5:1 to 25:1 in a further aspect, and from 4:1 to 15:1 in a still further aspect, wherein all ratios are calculated on a weight to weight basis (100 percent active material).

### Secondary Thickener- Component (c)

In one embodiment the surfactant-polymer blends of the invention include a secondary thickener selected from an esterified alkoxylated methyl glucoside. In one aspect of the invention, a suitable secondary thickener can be selected from an ethoxylated methyl glucose ether which has been esterified with a saturated or unsaturated C₁₀ to C₂₂ fatty acid(s). These fatty acid esters are prepared by ethoxylating the free hydroxyl groups of the methyl glucoside (e.g., methyl glucose) with ethylene oxide such that the total moles of ethoxylation can range from 100 to 150 in one aspect, from 110 to 135 in another aspect, and from 115 to 125 in a further aspect. Subsequent to the ethoxylation, esterification is effected with from 1.8 to 4 moles in one aspect, and from 2 to 3.5 moles of a saturated or unsaturated C₁₀ to C₂₂ fatty acid, and mixtures thereof. In one aspect, the fatty acid is selected from lauric, stearic, oleic, linoleic, linolenic, and mixtures thereof. In another aspect, the secondary thickener useful in optional combination with the surfactant-polymer blend(s) of the invention is commercially available under the INCI designation PEG-120 Methyl Glucose Dioleate containing 120 moles of ethoxylation and esterified with 2 moles of oleic acid. Such thickeners are commercially available under the Glucamate™ DOE-120 trade name marketed by Lubrizol Advanced Materials, Inc., Cleveland, Ohio.

It has been discovered that amino acid based surfactant compositions containing the hydrophobically-modified thickeners of the invention in combination with an esterified alkoxylated methyl glucoside exhibit a synergistic viscosity increasing effect when compared to amino acid surfactant compositions thickened with the hydrophobically-modified compositions only.

In one embodiment of the invention, the weight ratio of the hydrophobically-modified polymer thickener, component (b), to the optional secondary thickener, component (c), can be utilized in weight ratio ranging from 0.25:1 to 2:1 in one aspect, from 0.75:1 to 1.5:1 in another aspect, and from 1:1 to 1.2:1 in a further aspect, wherein all ratios are calculated on a weight to weight basis (100 percent active material).

In one embodiment, the amount of the optional secondary esterified alkoxylated methyl glucoside thickener that can be included in the personal care, household care, and industrial and institutional care compositions herein can range from 0 weight percent to 5 weight percent in one aspect, from 1.0 weight percent to 3.5 weight percent in another aspect, and from 1.75 weight percent to 2.5 weight percent in a further aspect of the invention, based on the total weight of the composition in which the optional secondary thickener is included. All weights are based on 100 percent of active material.

In one embodiment, a surfactant-polymer blend of the present invention can be added to one or more aqueous compositions (e.g., personal care and household care compositions) as a thickener. In one instance, a surfactant-polymer blend of this embodiment can be added at a concentration of 0.1 weight percent to 50 weight percent, based on 100 weight percent of the overall aqueous composition. In another embodiment, a surfactant-polymer blend of this embodiment can be added at a concentration of 0.5 weight percent to 45 weight percent in one aspect, from 1 weight percent to 40 weight percent in another aspect, from 5 weight percent to 35 weight percent in a further aspect, from 10 weight percent to 30 weight percent in a still further aspect, and from 15 weight percent to 25 weight percent in another aspect.

In one embodiment, a surfactant-polymer blend or a surfactant-polymer-secondary thickener blend of the present invention can be added to one or more aqueous compositions (e.g., personal care compositions, household care compositions, health care compositions) as a thickener at any concentration and/or weight range to yield a viscosity of at least 4,000 mPa·s (as measured after 24 hours with a Brookfield DV-II viscometer at 20 rpm, 25°C - Spindle #4, or sized as needed). In another embodiment, a surfactant-polymer blend of this embodiment can be added at any concentration and/or weight range to yield a viscosity of at least 5,000 mPa·s, at least 6,000 mPa·s, at least 7,500 mPa·s, at least 10,000 mPa·s, at least 12,500 mPa·s, at least 15,000 mPa·s, at least 17,500 mPa·s, at least 20,000 mPa·s, at least 25,000 mPa·s, at least 30,000 mPa·s, at least 35,000 mPa·s, at least 40,000 mPa·s, at least 45,000 mPa·s, at least 50,000 mPa·s, at least 55,000 mPa·s, at least 60,000 mPa·s, at least 65,000 mPa·s, at least 70,000 mPa·s, at least 75,000 mPa·s, at least 80,000 mPa·s, at least 85,000 mPa·s, at least 90,000 mPa·s, at least 95,000 mPa·s, or even at least 100,000 mPa·s.

As is discussed above, the surfactant-polymer blends and/or the surfactant-polymer-secondary thickener blends of the present invention can be added to any aqueous composition that are desired to be thickened. Such aqueous compositions include, but are not limited to, personal care products, household care products, health care products, institutional and industrial care products, and industrial applications.

The term "personal care products" as used herein includes, without being limited thereto, cosmetics, toiletries, cosmeceuticals and beauty aids, personal hygiene and cleansing products applied to the skin, hair, scalp, and nails of humans and animals. The term "health care products" as used herein includes, without being limited thereto, pharmaceuticals, pharmacosmetics, oral care products (mouth, teeth), eye care products, ear care products and over-the-counter products and appliances, such as patches, plasters, dressings and the like, and medical devices externally applied to or into the body of humans and animals for ameliorating a health-related or medical condition, for generally maintaining hygiene or well-being, and the like. The term "body" includes the keratinous (hair, nails) and non-keratinous skin areas of the entire body (face, trunk, limbs, hands and feet), the tissues of body openings and eyes, and the term "skin" includes the scalp and mucous membranes. The term "household care products" as used herein includes, without being limited thereto, products employed in a domestic household for surface cleaning or biocidal cleaning products for maintaining sanitary conditions, such as in the kitchen and bathroom, and laundry products for fabric care and cleaning, and the like. The term "institutional and industrial care" and "I&I," as used herein includes, without being limited thereto, products employed for cleaning or maintaining sanitary conditions in industrial and institutional environments, including hospital and health care facilities, and the like.

### Other Surfactant Components

In one embodiment, the surfactant-polymer components (a) and (b) of the present invention can be utilized in compositions containing one or more optional surfactants selected from anionic, cationic, amphoteric or zwitterionic, and nonionic surfactants. Any suitable combination of the aforementioned surfactants can be utilized as an optional surfactant component in the blends of the present invention.

### Anionic Surfactants

In one embodiment, the optional surfactant be selected from one or more suitable anionic surfactants in addition to components (a) and (b) described above. Such suitable anionic surfactants include, but are not limited to, one or more anionic cleansing surfactants which are cosmetically acceptable and suitable for topical application to the hair and skin. Examples of suitable anionic cleansing surfactants include, but are not limited to, alkyl sulphates, alkyl ether sulphates, alkaryl sulphonates, alkanoyl isethionates, alkyl succinates, alkyl sulphosuccinates, alkyl ether sulphosuccinates, alkyl phosphates, alkyl ether phosphates, and alkyl ether carboxylic acids and salts thereof (e.g., the sodium, magnesium, ammonium and mono-, di- and triethanolamine salts), or suitable mixtures of any two or more thereof. The alkyl groups contain, in one embodiment, from 8 to 18 carbon atoms, or even from about 10 to about 16 carbon atoms. In one embodiment, the alkyl and acyl groups can be unsaturated. The alkyl ether sulphates, alkyl ether sulphosuccinates, alkyl ether phosphates and alkyl ether carboxylic acids and salts thereof can contain from 1 to about 20, or even from 1 to 10, ethylene oxide or propylene oxide units per molecule. Here, as well as elsewhere in the specification and claims, individual numerical values, or limits, can be combined to form additional non-disclosed and/or non-stated ranges.

Typical anionic cleansing surfactants for use in personal care compositions (e.g., shampoos, body washes, etc.) of the invention include, but are not limited to, sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, and lauryl ether carboxylic acid.

In another embodiment, suitable anionic cleansing surfactants for use in conjunction with the blends of the present invention include, but are not limited to, sodium lauryl sulphate, sodium lauryl ether sulphate(c)EO, (where c is from 1 to 3), sodium lauryl ether sulphosuccinate(c)EO, (where c is from 1 to 3), ammonium lauryl sulphate, ammonium lauryl ether sulphate(c)EO, (where c is from 1 to 3), sodium cocoyl isethionate and lauryl ether carboxylic acid(c)EO (where c is from 10 to 20), or suitable mixtures of any two or more thereof.

In personal care (e.g., cleansing) compositions, the total amount of anionic cleansing surfactant can range from 0.5 weight percent to 45 weight percent in one aspect, from 1.5 weight percent to 35 weight percent in another aspect, and from 5 weight percent to 20 weight percent in a further aspect, based on the total weight of the composition. All weights are based on 100 percent of active material.

Shampoo compositions containing a blend according to one or more embodiments of the present invention can optionally comprise one or more anionic cleansing surfactants which are cosmetically acceptable and suitable for topical application to the hair and skin.

### Cationic Surfactants

In another embodiment, the optional surfactant is selected from one or more cationic surfactants in addition to components (a) and (b) described above. In one embodiment, personal care products (e.g., cleansing products with conditioners) that utilize a blend according to an embodiment of the present invention will typically comprise one or more cationic surfactants which are cosmetically acceptable and suitable for topical application to the hair or skin.

Examples of suitable cationic surfactants for use the blends of the present invention, where such blends are intended for use in personal care products (e.g., conditioners), include, but are not limited to, cetyltrimethylammonium chloride, behenyltrimethylammonium chloride, cetylpyridinium chloride, tetramethyl ammonium chloride, tetraethyl ammonium chloride, octyltrimethyl ammonium chloride, dodecyltrimethyl ammonium chloride, hexadecyltrimethyl ammonium chloride, octyldimethylbenzyl ammonium chloride, decyldimethylbenzyl ammonium chloride, stearyldimethylbenzyl ammonium chloride, didodecyldimethyl ammonium chloride, dioctadecyldimethyl ammonium chloride, tallowtrimethyl ammonium chloride, dihydrogenated tallow dimethyl ammonium chloride (e.g., Arquad 2HT/75 from Akzo Nobel), cocotrimethyl ammonium chloride, PEG-2-oleammonium chloride, the corresponding hydroxides thereof, or mixtures of two or more thereof. Other suitable cationic surfactants include, but are not limited to, those materials having the CTFA designations Quaternium-5, Quaternium-31 and Quaternium-18.

One cationic surfactant that is useful in personal care products (e.g., conditioners) in accordance with the present invention is cetyltrimethylammonium chloride which is available commercially from, for example, Hoechst Celanese as GENAMIN CTAC. Another cationic surfactant that is useful in personal care products (e.g., conditioners) in accordance with the present invention is behenyltrimethylammonium chloride which is available commercially from, for example, Clariant as GENAMIN KDMP.

Another example of a class of suitable cationic surfactants for use in the invention is a combination of compounds according to Formula (II) with an acid, where Formula (II) is an amidoamine:

R¹CONH(CH₂)_{b}N(R²)(R³) (II)

in which R¹ is a hydrocarbyl chain having 10 or more carbon atoms, R² and R³ are independently selected from hydrocarbyl chains of from 1 to 10 carbon atoms, and b is an integer from 1 to 10; and an acid.

As used herein, the term hydrocarbyl chain means an alkyl or alkenyl chain. In one embodiment, amidoamine compounds are those corresponding to Formula (I) in which R¹ is a hydrocarbyl residue having from 11 to 24 carbon atoms, R² and R³ are each independently hydrocarbyl residues, such as alkyl groups, having from 1 to 4 carbon atoms, and b is an integer from 1 to 4. In another embodiment, R² and R³ are methyl or ethyl groups. In still another embodiment, b is 2 or 3 (i.e., an ethylene or propylene group).

In one embodiment, amidoamines useful in conjunction with the present invention include, but are not limited to, stearamido-propyldimethylamine, stearamido-propyldiethylamine, stearamido-ethyldiethylamine, stearamido-ethyldimethylamine, palmitamido-propyldimethylamine, palmitamido-propyldiethylamine, palmitamido-ethyldiethylamine, palmitamido-ethyldimethylamine, behenamido-propyldimethylamine, behenamido-propyldiethylmine, behenamidoethyldiethylamine, behenamido-ethyldimethylamine, arachidamido-propyldimethylamine, arachidamido-propyldiethylamine, arachidamido-ethyldiethylamine, arachidamido-ethyldimethylamine, or mixtures of two or more thereof.

In still another embodiment, amidoamines useful in conjunction with the present invention include, but are not limited to, stearamido-propyldimethylamine, stearamido-ethyldiethylamine, or mixtures thereof.

Commercially available amidoamines useful herein include, but are not limited to, stearamido-propyldimethylamine sold under the tradenames LEXAMINE^{®}S-13 (available from Inolex of Philadelphia Pa., USA) and AMIDOAMINE® MSP (available from Nikko of Tokyo, Japan); stearamido-ethyldiethylamine sold under the tradename AMIDOAMINE^{®} S (available from Nikko of Tokyo, Japan); behenamido-propyldimethylamine sold under the tradename INCROMINE^{®} BB (available from Croda of North Humberside, England); and various amidoamines sold under the tradename SCHER-CODINE^{®} (available from Scher of Clifton N.J., USA).

The acid portion of the above-mentioned surfactant can be any organic or mineral acid which is capable of protonating the amidoamine in a personal care product (e.g., a hair treatment composition). Suitable acids for use in conjunction with the acid portion of the above-mentioned surfactant include, but are not limited to, hydrochloric acid, acetic acid, tartaric acid, fumaric acid, lactic acid, malic acid, succinic acid, or mixtures of two or more thereof. In one embodiment, the acid is selected from acetic acid, tartaric acid, hydrochloric acid, fumaric acid, or mixtures thereof.

The primary role of the acid is to protonate the amidoamine in the personal care product (e.g., a hair treatment composition) thus forming a tertiary amine salt (TAS) in situ in the personal care product. The TAS in effect becomes a non-permanent quaternary ammonium or pseudo-quaternary ammonium cationic surfactant. In one embodiment, the acid is included in a sufficient amount to protonate all the amidoamine present (i.e., at a level which is at least equimolar to the amount of amidoamine present in the composition). Mixtures of any of the above described cationic surfactants may also be used.

In two in one shampoo and conditioners and conditioning formulations of the kind covered within the scope of this invention, the level of cationic surfactant will generally range from 0.01 weight percent to 10 weight percent in one aspect, from 0.05 weight percent to 7.5 weight percent in another aspect, and from 0.1 weight percent to 5 weight percent in a further aspect, based on the total weight of the composition. All weights are based on 100 percent of active material.

In another embodiment, the present invention utilizes a combination of fatty alcohols and cationic surfactants in conditioning compositions is believed to be advantageous, because this leads to the formation of a lamellar phase, in which the cationic surfactant is dispersed. Representative fatty alcohols comprise from 8 to 22 carbon atoms or from 16 to 22. Fatty alcohols are typically compounds containing straight chain alkyl groups. Examples of suitable fatty alcohols include, but are not limited to, cetyl alcohol, stearyl alcohol, or mixtures of two or more thereof. The use of these materials is also advantageous in that they can contribute to the overall conditioning properties of compositions of the invention.

The level of fatty alcohol in the compositions of the present invention is in the range of from 0.01 weight percent to 10 weight percent in one aspect, from 0.1 weight percent to 8 weight percent in another aspect, from 0.2 weight percent to 7 weight percent in a further aspect, and from 0.3 weight percent to 6 weight percent in a still further aspect, based on the total weight of the composition. All weights are based on 100 percent of active material.

The weight ratio of cationic surfactant to fatty alcohol is in the range of from 1:1 to 1:10 in one aspect, from 1:1.5 to 1:8 in another aspect, and from 1:2 to 1:5, wherein all ratios are calculated on a weight to weight basis (100 percent active material). In one instance, if the weight ratio of cationic surfactant to fatty alcohol is too high, this can lead to eye irritancy from the composition. In another instance, if the weight ratio of cationic surfactant to fatty alcohol is too low, it can make the hair feel squeaky for some users.

### Amphoteric or Zwitterionic Surfactants

In one embodiment, the optional surfactant is selected from one or more amphoteric or zwitterionic surfactants such as alkylbetaines and alkylsulfobetaines are suitable for use in the blends of the present invention. In one embodiment, suitable amphoteric surfactants include, but are not limited to, alkyl betaines, alkyl amidopropyl betaines, cocoamidopropyl betaine (CAPB), cocobetaine, alkyl amine oxides, alkyl sulphobetaines (sultaines), alkyl amphoacetates, alkyl amphopropionates, alkyl amidopropyl hydroxysultaines, and acyl taurates, wherein the alkyl and acyl groups have from 8 to 19 carbon atoms. Typical amphoteric and zwitterionic surfactants for use in personal care (e.g., cleansing) compositions include, but are not limited to, lauryl amine oxide, cocodimethyl sulphopropyl betaine, lauryl betaine, cocamidopropyl betaine, lauroamphoacetate, and sodium cocoamphoacetate.

The amphoteric or zwitterionic surfactant(s), if present, can be included in an amount ranging from 0.5 weight percent to 8 weight percent in one aspect, from 1 weight percent to 4 weight percent in another aspect, based on the total weight of the composition to which a blend of the present invention is added. All weights are based on 100 percent of active material.

### Nonionic Surfactants

In another embodiment, the optional surfactant is selected from one or more nonionic surfactants in combination with components (a) and (b) described above. Suitable nonionic surfactants include, but are not limited to, alcohol ethoxylates, alkyl phenol ethoxylates, fatty acid alkanol amides, alkylamine oxides, N-methyl glucamides, alkyl polyglucosides, condensation products of aliphatic (C₈ to C₁₈) primary or secondary linear or branched chain alcohols or phenols with alkylene oxides, such as, for example ethylene oxide, and generally having from 2 to 30 ethylene oxide groups, coco mono- or di-ethanolamide and coco mono-isopropanolamide.

Additional nonionic surfactants which can be utilized in conjunction with in accordance with the present invention include, but are not limited to, alkyl polyglycosides (APGs). Typically, an APG is a compound which comprises an alkyl group connected (optionally via a bridging group) to a block of one or more glycosyl groups. Suitable APGs can be defined by Formula (I):

RO-(G)ₐ (I)

wherein R is a linear or branched alkyl group, a linear or branched alkenyl group, or a linear or branched alkynyl group; and G is a saccharide group. In one embodiment, R can be a linear or branched alkyl group having from 3 to 30 carbon atoms, or a linear or branched alkyl group having from 5 to 25 carbon atoms, or a linear or branched alkyl group having from 7 to 20 carbon atoms, or even a linear or branched alkyl group having from 8 to 15 carbon atoms. In still another embodiment, R is a branched or straight chain alkyl group having from about 9 to about 13 carbon atoms. In another embodiment, R can be a linear or branched alkenyl group having from 3 to 30 carbon atoms, or a linear or branched alkenyl group having from 5 to 25 carbon atoms, or a linear or branched alkenyl group having from 7 to 20 carbon atoms, or even a linear or branched alkenyl group having from 8 to 15 carbon atoms. In still another embodiment, R is a branched or straight chain alkenyl group having from 9 to 13 carbon atoms.

In still another embodiment, R can be a linear or branched alkynyl group having from 3 to 30 carbon atoms, or a linear or branched alkynyl group having from 5 to 25 carbon atoms, or a linear or branched alkynyl group having from 7 to 20 carbon atoms, or even a linear or branched alkynyl group having from 8 to 15 carbon atoms. In still another embodiment, R is a branched or straight chain alkynyl group having from 9 to 13 carbon atoms.

In one embodiment, G is selected from C₅ or C₆ monosaccharide residues, and is in one instance a glucoside. In another embodiment, G is selected from glucose, xylose, lactose, fructose, mannose, or derivatives thereof. In still another embodiment, G is glucose. In one embodiment, "a" is an integer in the range of from 1 to 10 in one aspect, 2 to 8 in another aspect, 3 to 7 in still another aspect, and from 4 to 6 in a further aspect. Here, as well as elsewhere in the specification and claims, individual numerical values, or limits, can be combined to form additional non-disclosed and/or non-stated ranges. In this embodiment, commercially available products are available under the trademark Plan-tacare^{®} and comprise a glucosidically bonded C₈ to C₁₆ alkyl group on an oligoglucoside residue whose average degree of oligomerization is 1 to 2. Exemplary alkyl glucosides and oligoglycosides are selected from octyl glucoside, decyl glucoside, lauryl glucoside, palmityl glucoside, isostearyl glucoside, stearyl glucoside, arachidyl glucoside and behenyl glucoside, and mixtures thereof.

Suitable alkyl polyglycosides for use in the invention are commercially available and include, but are not limited to, materials identified as: Oramix NS10 ex Seppic; Plantaren 1200 and Plantaren 2000 ex from Henkel. Other sugar-derived nonionic surfactants which can be included in compositions of the invention include, but are not limited to, C₁₀ to C₁₈ N-alkyl (C₁ to C₆) polyhydroxy fatty acid amides, such as the C₁₂ to C₁₈N-methyl glucamides, as described for example in WO 92/06154 and United States Patent No. 5,194,639, and N-alkoxy polyhydroxy fatty acid amides, such as C₁₀ to C₁₈ N-(3-methoxypropyl) glucamide.

The nonionic surfactant(s), if present, can be included in an amount ranging 0.1 weight percent to 12 weight percent in one aspect, from 0.5 weight percent to 8 weight percent in another aspect, from 1 weight percent to 6 weight percent in still another aspect, and from 2 weight percent to 5 weight percent in a further aspect, based on the total weight of the composition to which the blend of the present invention is added. All weights are based on 100 percent of active material.

As previously stated the surfactant-polymer components (a) and (b) of the present invention can be utilized in compositions containing one or more of the foregoing optional surfactants selected from anionic, cationic, amphoteric or zwitterionic, and nonionic surfactants. Any suitable combination of the aforementioned surfactants can be utilized as an optional surfactant component in the blends of the present invention. In one embodiment, the total amount of surfactant included in the compositions herein can range of from 1 weight percent to 50 weight percent in one aspect, or from 2 weight percent to 40 weight percent in another aspect, and from 10 weight percent to 25 weight percent in a further aspect, based on the total weight of the composition to which a blend of the present invention and optional surfactants are added. Again, all weights are based on 100 percent of active material.

### Optional Components

In still another embodiment, the blends of the present invention can include one or more optional components that are typically found in personal care products, health care products, household care products, institutional and industrial care products, and industrial applications. Such optional components include, but are not limited to, one or more cationic polymers, one or more multipurpose polymers, one or more compounds that enhance performance and/or consumer acceptability, or mixtures of two or more thereof. Such one or more compounds that enhance performance and/or consumer acceptability include fragrance, dyes and pigments, pH adjusting agents (acids and/or bases), pearlizing agents or opacifiers, other viscosity modifiers, preservatives, and natural hair nutrients such as botanicals, fruit extracts, sugar derivatives, solvents and amino acids.

### Cationic Polymers

In one embodiment, the optional cationic polymers that can be utilized in conjunction with the blends of the present invention can be homopolymers which are cationically substituted or can be formed from two or more types of monomers. The weight average (M_{w}) molecular weight of the polymers are in the range of 75,000 daltons to 2,500,000 daltons in one aspect, and from 100,000 daltons to 2,000,000 daltons in another aspect.

In one embodiment, the optional cationic polymers that can be utilized in conjunction with the blends of the present invention have cationic nitrogen-containing groups such as quaternary ammonium or protonated amino groups, or a mixture thereof. In one personal care embodiment, if the molecular weight of the polymer is too low, then the conditioning effect is poor. If too high, then there can be problems of high extensional viscosity leading to stringiness of the composition when it is poured.

In one embodiment, the cationic nitrogen-containing group will generally be present as a substituent on a fraction of the total monomer units of the cationic polymer. Thus, in embodiments where the cationic polymer is not a homopolymer it can contain spacer non-cationic monomer units. Such polymers are described in the CTFA Cosmetic Ingredient Directory, 3rd Edition. In one embodiment, the ratio of the cationic to non-cationic monomer units is selected to give polymers having a cationic charge density in the required range, which in one embodiment is from 0.2 to 3.0 meq/gm. The cationic charge density of the polymer is suitably determined via the Kjeldahl method as described in the US Pharmacopoeia under chemical tests for nitrogen determination.

In one embodiment, suitable cationic polymers include, but are not limited to, copolymers of vinyl monomers having cationic amine or quaternary ammonium functionalities with water soluble spacer monomers such as (meth)acrylamide, alkyl and dialkyl(meth)acrylamides, alkyl(meth)acrylate, vinyl caprolactone and vinyl pyrrolidine. The alkyl and dialkyl substituted monomers can contain C₁ to C₇ alkyl groups, or even C₁ to C₃ alkyl groups. Other suitable spacers include vinyl esters, vinyl alcohol, maleic anhydride, propylene glycol and ethylene glycol.

In one embodiment, the cationic amines can be primary, secondary or tertiary amines, depending upon the particular species and the pH of the composition. In another embodiment, secondary and tertiary amines, or even tertiary amines are utilized. Amine substituted vinyl monomers and amines can be polymerized in the amine form and then converted to ammonium by quaternization. In still another embodiment, the cationic polymers can comprise mixtures of monomer units derived from amine- and/or quaternary ammonium-substituted monomer and/or compatible spacer monomers.

Suitable cationic polymers include, but are not limited to, cationic diallyl quaternary ammonium-containing polymers including, but not limited to, dimethyldiallyl ammonium chloride homopolymer and copolymers of acrylamide and dimethyldiallyl ammonium chloride, referred to in the industry (CTFA) as Polyquaternium 6 and Polyquaternium 7, respectively; mineral acid salts of amino-alkyl esters of homo- and co-polymers of unsaturated carboxylic acids having from about 3 to about 5 carbon atoms (as described in United States Patent No. 4,009,256; mixtures of two or more thereof.

Other cationic polymers that can be used in conjunction with the blends of the present invention include, but are not limited to, cationic polysaccharide polymers, such as cationic cellulose derivatives, cationic starch derivatives, and cationic guar gum derivatives. Cationic polysaccharide polymers suitable for use in compositions of the invention include, but are not limited to, monomers having the Formula shown below:

E-O-[R₁₀-N⁺(R₁₁)(R₁₂)(R₁₃)X₃]

where E is an anhydroglucose residual group, such as a starch or cellulose anhydroglucose residual; R₁₀ is an alkylene, oxyalkylene, polyoxyalkylene, or hydroxyalkylene group, or combination thereof; R₁₁, R₁₂, and R₁₃ are independently selected from alkyl, aryl, alkylaryl, arylalkyl, alkoxyalkyl, or alkoxyaryl groups, where each group can independently contain from up to 18 carbon atoms. The total number of carbon atoms for each cationic moiety (i.e., the sum of carbon atoms in R₁₁, R₁₂ and R₁₃) is 54 or less, 40 or less, 30 or less, or even 20 or less; and X₃ is an anionic counter-ion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation under, for example, the tradename Polymer LM-200. Other suitable cationic polysaccharide polymers include quaternary nitrogen-containing cellulose ethers (e.g., those described in United States Patent No. 3,962,418), and copolymers of etherified cellulose and starch (e.g., those described in United States Patent No. 3,958,581), or mixtures of two or more thereof.

A particularly suitable type of cationic polysaccharide polymer that can be used is a cationic guar gum derivative, such as guarhydroxypropyltrimethyl ammonium chloride (commercially available from Rhodia in their JAGUAR^{®} series). Examples of such materials include, but are not limited to, JAGUAR^{®} C13S, JAGUAR^{®} C14, JAGUAR^{®} C15, JAGUAR^{®} C17, JAGUAR^{®} C16, JAGUAR^{®} CHT, and JAGUAR^{®} C162.

In still another embodiment, mixtures of any of the above cationic polymers can be used. If present, the one or more cationic polymers are present in a personal care composition (e.g., a shampoo composition) at levels of from about 0.01 weight percent to about 5 weight percent, or from 0.05 weight percent to about 1 weight percent, or even from 0.08 weight percent to about 0.5 weight percent, based on the total weight of the composition.

### Applications for the Surfactant-Polymer Blends

As used herein the term "blend" or "blends" refers to the surfactant-polymer blends of the present invention regardless of whether or not such blends contain one or more salts compounds disclosed herein. As such, the terms "blend," "blends," "surfactant-polymer blend," or "surfactant-polymer blends" are meant to encompass all of the embodiments of the present invention as disclosed herein and are in no way meant to be limiting in scope.

The surfactant-polymer blends compositions of the invention can be utilized in a variety of products for personal care, health care, home care, institutional and industrial (collectively "I&I") care, and in a variety of products for medical and industrial applications. The inventive blends of the present invention can be employed as emulsifiers, stabilizers, suspending agents, deposition aids for enhancing the efficacy, deposition or delivery of chemically and physiologically active ingredients and cosmetic materials, film formers, thickeners, rheology modifiers, hair fixatives, conditioners, moisturizers, spreading aids, carriers, and as an agent for improving the psychosensory, and aesthetic properties of a formulation in which they are included.

### Personal Care and Health Care Applications

The surfactant-polymer blends of the present invention are, in one embodiment, suitable for the preparation of personal care (e.g., cosmetics, toiletries, cosmeceuticals), topical health care products and medical products including, but not limited to, hair care products, such as shampoos (including combination shampoos, such as "two-in-one" conditioning shampoos and anti-dandruff shampoos); post-shampoo rinses; setting and style maintenance agents including setting aids and fixatives, such as gels and sprays, grooming aids, such as pomades, conditioners, perms, relaxers, hair smoothing products, and the like; skin care products (e.g., facial, body, hands, scalp and feet), such as creams, lotions, conditioners, deodorants, antiperspirants and cleansing products; anti-acne products; anti-aging products (e.g., exfoliant, keratolytic, anti-cellulite, anti-wrinkle, and the like); skin protectants such as sunscreens, sunblock, barrier creams, oils, silicones, and the like; skin color products (e.g., whiteners, lighteners, sunless tanning accelerators, and the like); hair colorants (e.g., hair dyes, hair color rinses, highlighters, bleaches and the like); pigmented skin colorants (e.g., face and body make-ups, foundation creams, mascara, rouge, lip products, and the like); bath and shower products (e.g., body cleansers, body washes, shower gels, liquid soaps, soap bars, syndet bars, conditioning liquid bath oil, bubble bath, bath powders, therapeutic cleaners, acne control products, facial cleansers and the like); nail care products (e.g., polishes, polish removers, strengtheners, lengtheners, hardeners, cuticle removers, softeners, and the like); and any aqueous composition to which an effective amount of surfactant-polymer blend composition can be incorporated for achieving a beneficial or desirable, physical or chemical effect therein during storage and/or usage.

In one aspect the surfactant-polymer blends of the present invention can be used as a thickener, rheology modifier, film former, deposition aid, or as a dye or pigment suspending agent for promoting deposition of colorants on hair and skin. Colorants for hair can be temporary, semi-permanent or permanent hair dyes or color restorers that pigment the hair gradually. Temporary and semi-permanent hair dyes typically are rinses, gels, sprays, shampoos, sticks, and the like, and hair color restorers are typically in the form of hair dressings or emulsions. Permanent hair dyes, and longer-lasting semi-permanent hair dyes, are generally two-part products, one part containing the oxidative dye intermediates and dye couplers, and the other part containing stabilized oxidizing agent, usually hydrogen peroxide at pH 3 to 4, and are mixed together immediately before use. It is known that such two-part hair dyeing products are formulated with combinations of surfactant ingredients, usually nonionic surfactants or anionic surfactants, to thicken when the dye mixture is prepared. In addition to the foregoing literature, a general discussion of hair dyeing chemistry and compositions is in Brown et al, SCC Monograph, Permanent Hair Dyes, Society of Cosmetic Chemists (1996). The polymers of the present invention may be incorporated in one or both of the two-parts of such hair dyeing systems, either as the thickener for the acidic stabilized oxidizing portion or in the non-oxidizing portion to be thickened upon mixing with the acidic portion.

In another hair care embodiment, the inventive surfactant-polymer blends can be utilized in an amount effective to provide to the hair care composition a property, such as a hair fixative property, a hair conditioning property, a viscid property (thickening or rheology modifying), or a combination thereof. Optionally, the hair care composition can include one or more auxiliary film-forming agents, auxiliary hair fixative agent, auxiliary hair conditioning agent, auxiliary rheology modifying agent, or mixtures thereof.

The term "fixative" encompasses the properties of film-formation, adhesion, or a coating deposited on a surface on which the polymer is applied. The terms "hair setting," "hair styling" and "hair fixative" as commonly understood in the hair care arts, and as used herein, refer collectively to hair setting agents that are hair fixatives and film formers and which are topically applied to the hair to actively contribute to the ease of styling and/or holding of a hair set, and to maintain the restylability of the hair set. Hence, "hair setting compositions" include hair styling, hair fixative, and hair grooming products that conventionally are applied to the hair (wet or dry) in the form of gels, rinses, emulsions (oil-in-water, water-in-oil or multiphase), such as lotions and creams, pomades, sprays (pressurized or non-pressurized), spritzes, foams, such as mousses, shampoos, solids, such as sticks, semisolids and the like, or are applied from a hair setting aid having the hair setting composition impregnated therein or coated thereon, to leave the hair setting agent in contact on the hair for some period until removed, as by washing.

The term "hair setting composition" encompasses products comprising at least one surfactant-polymer blend of the present invention as a hair setting agent or as an adjuvant in combination with an auxiliary hair fixative agent, which is applied to the hair (wet or dry) before, during or after configuring the hair into the shape (curly or straight) desired, without limitation as to product form.

The term "conditioning agents," and grammatical variations thereof, as it relates to compositions for skin care and hair care includes cosmetically and pharmaceutically useful materials that are humectants, moisturizers, and emollients. It is recognized that some conditioning agents can serve more than one function in a composition, such as emulsifying agents, lubricants, and solvents.

The surfactant-polymer blends of the present invention are surprisingly useful in hair setting and hair styling compositions as the sole film-forming, rheology modifying, conditioning, and fixative agent. The surfactant-polymer blends of the present invention are also useful in combination with commercially available auxiliary hair fixative polymers, such as nonionic, cationic, and amphoteric hair setting polymers, cationic conditioning polymers, and combinations thereof. It is surprisingly found that unexpectedly increased viscosity and hair setting efficacy properties are produced by appropriate combinations of a polymer of the present invention with an auxiliary conventional hair fixative and/or hair conditioning polymer. Conventional polymeric hairfixative and hair styling polymers, well known in the art, include natural gums and resins and neutral or anionic polymers of synthetic origin. Listings of commercially available hair fixative and conditioning fixative polymers can be readily found in the INCI Dictionary, in supplier websites, and in the trade literature. See, for example, the Polymer Encyclopedia published in Cosmetics & Toiletries®, 117(12), December 2002 (Allured Publishing Corporation, Carol Stream, III.).

Suitable commercially available auxiliary fixatives include, but are not limited to, nonionic, cationic, anionic, and amphoteric polymers, as well as combinations thereof and include without limitation, polyvinylpyrrolidone (PVP), polyvinyl-pyrrolidone/vinylacetate copolymer (PVP/VA), and the like. Commercially available cationic fixative polymers include, without limitation thereto, polymers having the INCI name, polyquaternium, such as polyquaternium-4, a diallyldimonium chloride/hydroxy-ethylcellulose copolymer (such as CELQUAT^{®} H-100, National Starch); polyquaternium-11, a quaternized vinyl pyrrolidone/dimethylaminoethyl methacrylate copolymer (such as GAFQUAT^{®} 734, 755, 755N, ISP); polyquaternium-16, a quaternized vinyl pyrrolidone/vinylimidazolium chloride copolymer (such as LUVIQUAT^{®} FC-370, BASF); polyquaternium-28, a vinylpyrrolidone/methacryl-amidopropyltrimethylammonium chloride copolymer(such as GAFQUAT^{®} HS-100, ISP); polyquaternium-46, a quaternized vinylcaprolactam/vinylpyrrolidone/methylvinyl-imidazolium methosulfate copolymer; polyquaternium-55, a quaternized vinyl-pyrrolidone/dimethylamino-propylmethylacrylamide/lauryldimethylpropylmethacrylamido-ammonium chloride copolymer (such as STYLEZE^{™} W, ISP), and the like; and amino-substituted polymers which are cationic under acidic pH conditions, such as vinylcaprolactam/PVP/dimethylaminoethylmethacrylate copolymer (such as GAFFIX^{®} VC-713, ISP); PVP/dimethylaminoethylmethacrylate copolymer (such as Copolymer 845, ISP), PVP/DMAPA acrylates copolymer (such as STYLEZE^{™} CC-10, ISP), the pyrrolidone carboxylic acid salt of chitosan, having the INCI name, Chitosan PCA (such as KYTAMER^{®} PC, Amerchol), and the like.

Additional auxiliary fixatives can be selected from one or more of the following polymers. Suitable commercially available nonionic polymers (i.e., neutral) used as hair styling or fixative polymers include, without limitation thereto, polyvinylpyrrolidone (PVP), polyvinylpyrrolidone/vinylacetate copolymer (PVP/VA), and the like. Commercially available cationic fixative polymers include, without limitation thereto, polymers having the INCI name, polyquaternium, such as polyquaternium-4, a diallyldimonium chloride/hydroxyethylcellulose copolymer (such as CELQUAT^{®} H-100, National Starch); polyquaternium-11, a quaternized vinyl pyrrolidone/dimethylaminoethyl methacrylate copolymer (such as GAFQUAT^{®} 734, 755, 755N, ISP); polyquaternium-16, a quaternized vinyl pyrrolidone/vinylimidazolium chloride copolymer (such as LUVIGUAT^{®} FC-370, BASF); polyquaternium-28, a vinylpyrrolidone/methacryl-amidopropyltrimethylammonium chloride copolymer (such as GAFQUAT^{®} HS-100, ISP); polyquaternium-46, a quaternized vinylcaprolactam/vinylpyrrolidone/methylvinyl-imidazolium methosulfate copolymer; polyquaternium-55, a quaternized vinyl-pyrrolidone/dimethylaminopropylmethylacrylamide/lauryldimethylpropylmethacryl amido-ammonium chloride copolymer (such as STYLEZE^{™} W, ISP), and the like; and amino-substituted polymers which are cationic under acidic pH conditions, such as vinylcaprolactam/PVP/dimethylaminoethylmethacrylate copolymer (such as GAFFIX^{®} VC-713, ISP); PVP/dimethylaminoethylmethacrylate copolymer (such as Copolymer 845, ISP), PVP/DMAPA acrylates copolymer (such as STYLEZE^{™} CC-10, ISP), the pyrrolidone carboxylic acid salt of chitosan, having the INCI name, Chitosan PCA (such as KYTAMER^{®} PC, Amerchol), and the like. Suitable amphoteric fixative polymers include, without limitation thereto, octylacryamide/acrylates/butylaminoethylmethacrylate copolymer (such as the AMPHOMER^{®} polymers, National Starch), acrylates/lauryl acrylate/stearyl acrylate/ethylamine oxide methacrylate copolymers (such as the DIAFORMER^{®} polymers, Clariant Corp.), and the like. acrylamide/acrylates/butyl-aminoethylmethacrylate copolymer (such as the AMPHOMER^{®} polymers, National Starch), acrylates/lauryl acrylate/stearyl acrylate/ethylamine oxide methacrylate copolymers (such as the DIAFORMER^{®} polymers, Clariant Corp.), and the like.

Additional fixative polymers that can be utilized with the inventive polymers of the invention or in combination with one or more of the foregoing auxiliary fixatives, include without limitation, one or more of the following fixative polymers: polyoxythylenated vinyl acetate/crotonic acid copolymers, vinyl acetate crotonic acid copolymers, vinyl methacrylate copolymers, monoalkyl esters of poly(methyl vinyl ether (PVM)/maleic acid (MA)), such as, for example, ethyl, butyl and isopropyl esters of PVM/MA copolymer acrylic acid/ethyl acrylate/N-tert-butyl-acrylamide terpolymers, and poly (methacrylic acid/acrylamidomethyl propane sulfonic acid), acrylates copolymer, acrylates/octylacrylamide copolymer, vinyl acetate (VA)/crotonates/vinyl neodecanoate copolymer, poly(N-vinyl acetamide), poly(N-vinyl formamide), corn starch modified, sodium polystyrene sulfonate, polyquaterniums such as, for example, Polyquaternium-24, Polyquaternium-29, Polyquaternium-32, Polyquaternium-34, Polyquaternium-37, Polyquaternium-39, Polyquaternium-44, Polyquaternium-47, Polyquaternium-69, Polyquaternium-87, polyether-1, polyurethanes, VA/acrylates/lauryl methacrylate copolymer, adipic acid/dimethylaminohydroxypropyl diethylene AMP/acrylates copolymer, methacrylol ethyl betaine/acrylates copolymer, VP/methacrylamide/vinyl imidazole copolymer, VP/vinylcaprolactam/DMAPA acrylates copolymer, VP/dimethylaminoethylmethacrylate copolymer, VP/DMAPA acrylates copolymer, vinyl caprolactam/VP/dimethylaminoethyl methacrylate copolymer, VA/butyl maleate/isobornyl acrylate copolymer, VA/crotonates copolymer, acrylate/acrylamide copolymer, VA/crotonates/vinyl propionate copolymer, VP/vinyl acetate/vinyl propionate terpolymers, VA/crotonates, VP/vinyl acetate copolymer, VP/acrylates copolymer, VA/crotonic acid/vinyl propionate, acrylates/acrylamide, acrylates/octylacrylamide, acrylates/hydroxyacrylates copolymer, acrylates/hydroxyesteracrylates copolymer, acrylates/stereth-20 methacrylate copolymer, tert-butyl acrylate/acrylic acid copolymer, diglycol/cyclohexanedimethanol/isophthalates/sulfoisophthalates copolymer, VA/butyl maleate and isobornyl acrylate copolymer, vinylcaprolactam/VP/dimethylaminoethyl methacrylate, VA/alkylmaleate half ester/N-substituted acrylamide terpolymers, vinyl caprolactam/VP/ methacrylamidopropyl trimethylammonium chloride terpolymer, methacrylates/acrylates copolymer/amine salt, polyvinylcaprolactam, polyurethanes, hydroxypropyl guar, poly (methacrylic acid/acrylamidomethyl propane sulfonic acid (AMPSA), ethylenecarboxamide (EC)/AMPSA/methacrylic acid (MAA), poylurethane/acrylate copolymers and hydroxypropyl trimmonium chloride guar, acrylates copolymer, acrylates crosspolymer, AMP-acrylates/allyl methacrylate copolymer, polyacrylate-14, polyacrylate-2 crosspolymer, octylacryl-amide/acrylates/butylaminoethyl methacrylate copolymer, acrylates/octylacrylamide copolymer, VA/crotonates/vinyl neodecanoate copolymer, poly(N-vinyl acetamide), poly(N-vinyl formamide), polyurethane, methacryloyl ethyl betaines/methacrylates copolymer, corn starch modified, sodium polystyrene sulfonate, polyurethane/acrylates copolymer, chitosan glycolate, cationic polygalactomannans, such as, for example, quaternized derivatives of guar and cassia, such as, for example, guar hydroxypropyl trimmonium chloride, hydroxypropyl guar hydroxypropyl trimmonium chloride and cassia hydroxypropyl trimmonium chloride. Many of the foregoing polymers are referred to by their INCI nomenclature set forth in the International Cosmetic Ingredient Dictionary published by the Cosmetic, Toiletry, and Fragrance Association, Washington D.C. Other suitable auxiliary fixative polymers are disclosed in United States Patent No. 7,205,271.

Toiletries and health and beauty aids, commonly referred to as HBAs, containing a surfactant-polymer blend of the invention, can include, but are not limited to, hair-removal products (e.g., shaving creams and lotions, depilatories, after-shave skin conditioners, and the like); deodorants and antiperspirants; oral care products (e.g., for the mouth, teeth and gums), such as mouthwash, dentifrice, such as toothpaste, tooth powder, tooth polishes, tooth whiteners, breath fresheners, denture adhesives, and the like; facial and body hair bleach; and the like. Other health and beauty aids that can contain surfactant-polymer blends of the present invention include, but are not limited to, sunless tanning applications containing artificial tanning accelerators, such as dihydroxyacetone (DHA), tyrosine, tyrosine esters, and the like; skin de-pigmenting, whitening, and lightening formulations containing such active ingredients as kojic acid, hydroquinone, arbutin, fruital, vegetal or plant extracts, (lemon peel extract, chamomile, green tea, paper mulberry extract, and the like), ascorbyl acid derivatives (ascorbyl palmitate, ascorbyl stearate, magnesium ascorbyl phosphate, and the like); foot care products, such as keratolytic corn and callous removers, foot soaks, foot powders (medicated, such as antifungal athlete's foot powder, ointments, sprays, and the like, and antiperspirant powders, or non-medicated moisture absorbent powder), liquid foot sprays (non-medicated, such as cooling, and deodorant sprays, and medicated antifungal sprays, antiperspirant sprays, and the like), and foot and toenail conditioners (lotions and creams, nail softeners, and the like).

Topical health and beauty aids that can include the surfactant-polymer blends of the invention (e.g., thickeners, rheology modifiers, spreading aids, deposition aids and film formers) include, but are not limited to, skin protective spray, cream, lotion, gel, stick and powder products, such as insect repellants, itch relief, antiseptics, disinfectants, sun blocks, sun screens, skin tightening and toning milks and lotions, wart removal compositions, and the like.

Other health care products in which surfactant-polymer blends can be included are medical products, such as topical and non-topical pharmaceuticals, and devices. In the formulation of pharmaceuticals, a surfactant-polymer blend of the invention can be employed as a thickener or rheology modifier in such products as creams, pomades, gels, pastes, ointments, tablets, gel capsules, purgative fluids (e.g., enemas, emetics, colonics, and the like), suppositories, antifungal foams, eye products (e.g., ophthalmic products, such as eye-drops, artificial tears, glaucoma drug delivery drops, contact lens cleaner, and the like), ear products (e.g., wax softeners, wax removers, otitis drug delivery drops, and the like), nasal products (e.g., drops, ointments, sprays, and the like), and wound care (e.g., liquid bandages, wound dressings, antibiotic creams, ointments, and the like), without limitation thereto.

The surfactant-polymer blends are useful as thickeners, rheology modifiers, deposition aids, and film-formers in a variety of dermatological, pharmaceutical, and cosmeceutical compositions employed for topically ameliorating skin and scalp conditions caused by perspiration, inflammation, swelling, drying, dandruff, photo-damage, aging, acne, and the like, containing pharmaceutical and cosmeceutical active ingredients, conditioners, surfactants, moisturizers, antioxidants, exfoliants (described above), keratolytic agents, botanicals, vitamins, and the like, and combinations thereof.

The film-forming character of compositions that utilize the surfactant-polymer blends of the present invention make compositions containing a blend of the present invention particularly suitable as a vehicle for topical medical compositions for promoting and enhancing the transdermal delivery of active ingredients to or through the skin, for enhancing the efficacy of anti-acne agents formulations and topical analgesics, and for controlling release of drugs, such as antacids from tablets, or syrups; controlling drug release from tablets, lozenges, chewables, and the like in the mildly acidic environment of the mouth; or from suppositories, ointments, creams, and the like in the mildly acidic environment of the vagina; to promote deposition of dandruff control agents from shampoos, salves, and the like; to enhance the deposition of colorants on skin from pigmented cosmetics (e.g., make-ups, lipsticks, rouges, and the like) and on hair from hair dyes, and the like.

Suitable examples of pharmaceutical and cosmeceutical active ingredients include, but are not limited to, caffeine, vitamin C, vitamin D, vitamin E, pantothenic acid (vitamine B5), anti-stretch mark compounds, astringents (e.g., alum, oatmeal, yarrow, witch hazel, bayberry, and isopropyl alcohol), draining compounds, hair growth compounds (e.g., monoxidil), skin and hair nourishing compounds, skin and hair protecting compounds, self-tanning compounds (e.g., mono- or polycarbonyl compounds such as, for example, isatin, alloxan, ninhydrin, glyceraldehyde, mesotartaric aldehyde, glutaraldehyde, erythrulose, tyrosine, tyrosine esters, and dihydroxyacetone), sunscreens (e.g., ethylhexyl methoxy cinnamate, octinoxate, octisalate, oxybenzone), skin lighteners (e.g., kojic acid, hydroquinone, arbutin, fruital, vegetal or plant extracts, such as lemon peel extract, chamomile, green tea, paper mulberry extract, and the like, ascorbyl acid derivatives, such as ascorbyl palmitate, ascorbyl stearate, magnesium ascorbyl phosphate, and the like), lip plumping compounds, anti-aging, anti-cellulite, and anti-acne compounds (e.g., acidic agents such as alpha-hydroxy acids (AHAs), beta-hydroxy acids (BHAs), alpha amino-acids, alpha-keto acids (AKAs), acetic acid, azelaic acid, and mixtures thereof), anti-dandruff compounds (e.g., zinc pyrithione, zinc omadine, miconazole nitrate, selenium sulfide, piroctone olamine) anti-inflammatory compounds (e.g., aspirin, ibuprofen, and naproxen), analgesics (e.g., acetaminophen), antioxidant compounds, antiperspirant compounds, deodorant compounds (e.g., 2-amino-2-methyl-1-propanol (AMP), ammonium phenolsulfonate; benzalkonium chloride; benzethonium chloride, bromochlorophene, cetyltrimethylammonium bromide, cetyl pyridinium chloride, chlorophyllin-copper complex, chlorothymol, chloroxylenol, cloflucarban, dequalinium chloride, dichlorophene, dichloro-m-xylenol, disodium dihydroxyethyl sulfosuccinylundecylenate, domiphen bromide, hexachlorophene, lauryl pyridinium chloride, methylbenzethonium chloride, phenol, sodium bicarbonate, sodium phenolsulfonate, triclocarban, triclosan, zinc phenolsulfonate, zinc ricinoleate, and mixtures thereof), hair fixative polymers (e.g., natural and synthetic polymers such as, for example, polyacrylates, polyvinyls, polyesters, polyurethanes, polyamides, modified cellulose, starches, and mixtures thereof), hair and skin conditioners (e.g., synthetic oils, natural oils, such as vegetable, plant and animal oils, mineral oils, natural and synthetic waxes, cationic polymers, monomeric and polymeric quaternized ammonium salt compounds, silicones such as silicone oils, resins and gums, proteins, hydrolyzed proteins, fatty acids, fatty amines; and mixtures thereof); and suitable mixtures of two or more of the above.

In one cosmeceutical aspect, a surfactant-polymer blend can be employed as a thickener, rheology modifier and/or a deposition aid for active skin treatment lotions and creams containing, as active ingredients, acidic anti-aging, anti-cellulite, and anti-acne agents, hydroxy carboxylic acids, such as alpha-hydroxy acid (AHA), beta-hydroxy acid (BHA), alpha-amino acid, alpha-keto acids (AKAs), and mixtures thereof. Suitable AHAs include, but are not limited to, lactic acid, glycolic acid, fruit acids, such as malic acid, citric acid, tartaric acid, extracts of natural compounds containing AHA, such as apple extract, apricot extract, and the like, honey extract, 2-hydroxyoctanoic acid, glyceric acid (dihydroxypropionic acid), tartronic acid (hydroxypropanedioic acid), gluconic acid, mandelic acid, benzilic acid, alpha-lipioc acid, AHA salts and derivatives, such as arginine glycolate, ammonium lactate, sodium lactate, alpha-hydroxybutyric acid, alpha-hydroxyisobutyric acid, alpha-hydroxyisocaproic acid, alpha-hydroxyisovaleric acid, atrolactic acid, and the like. Suitable BHAs include, but are not limited to, 3-hydroxy propanoic acid, beta-hydroxybutyric acid, beta-phenyl lactic acid, beta-phenylpyruvic acid, salicylic acid, and the like. Suitable alpha-amino acids include, but are not limited to, alpha-amino dicarboxylic acids, such as aspartic acid, glutamic acid, and the like. Representative alpha-keto acids are pyruvic acid, acetopyruvic acid, and the like. Other active acidic agents include retinoic acid and its derivatives, halocarboxylic acids (e.g., trichloroacetic acid), acidic antioxidants (e.g., vitamin C), mineral acids, phytic acid, lysophosphatidic acid, salicylic acid, derivatives of salicylic acid (e.g., 5-octanoylsalicylic acid), and the like. Typically the active acidic ingredient has a pH in the range of 0.5 to 5.

A discussion of the use and formulation of active skin treatment compositions is in COSMETICS & TOILETRIES®, C&T Ingredient Resource Series, AHAs & Cellulite Products How They Work, published 1995, and Cosmeceuticals, published 1998, both available from Allured Publishing Corporation. Compositions containing alpha-amino acids acidified with ascorbic acid are described in United States Patent No. 6,197,317 and United States Patent Application Publication No. 2009/0029928, and a commercial cosmeceutical preparation utilizing these acids in an anti-aging, skin care regimen is sold under the tradename, AFAs, by exCel Cosmeceuticals (Bloomfield Hills, Michigan). The term "AFA," as described in the supplier's trade literature was coined by the developer to describe the amino acid/vitamin C combination as Amino Fruit Acids and as the acronym for "Amino acid Filaggrin based Antioxidants." In addition to ingredients discussed above, other ingredients commonly used for anti-acne products, facial and body hair bleaches, and anti-septic products include oxidizing agents, such as hydrogen peroxide, benzoyl peroxide, and water-soluble inorganic persulfate compounds such as ammonium persulfate, potassium persulfate, and sodium persulfate.

The surfactant-polymer blends of the present invention can be employed, without being limited thereto, as a lubricant coating for medical devices, such as soft tissue implants, surgical gloves, catheters, cannulae, and the like, as removable protective film coatings for medical instruments, wound dressings, and the like, as a muco-adhesive, especially in the acid environment of the stomach, as a carrier and thickener in formulated products for medical applications, such as disinfectant hand creams, antiviral products, antibiotic ointments, sprays and creams, non-drip, sprayable disinfectant in hospitals, hard surface antimicrobial finish applied during routine maintenance, and the like.

### Home Care and Institutional and Industrial Applications

The surfactant-polymer blends of the present invention can be used in home care, and I&I applications, for example, as a thickener or rheology modifier, to improve formulation efficiency through "cling-on-surface" or for improving the efficacy of disinfectants, antistatic agents, optional detersive surfactants, and biocidal formulations, and to synergistically improve fabric softening efficacy in combination with traditional fabric softeners. Typical household and I&I products that may contain polymers of the invention include, but are not limited to, laundry and fabric care products, such as detergents, laundry pre-spotting removal products, fabric softeners (liquids or sheets), ironing sprays, dry cleaning aids, anti-wrinkle sprays, spot removers and the like; hard surface cleansers for the kitchen and bathroom and utilities and appliances employed or located therein, such as toilet bowl gels, tub and shower cleaners, hard water deposit removers, floor and tile cleansers, wall cleansers, floor and chrome fixture polishes, alkali-strippable vinyl floor cleaners, marble and ceramic cleaners, air freshener gels, liquid cleansers for dishes, and the like; disinfectant cleaners, such as toilet bowl and bidet cleaners, disinfectant hand soaps, room deodorizers, and the like.

Antistatic agents include, but are not limited to, quaternary ammonium compounds, protein derivatives, synthetic quaternary ammonium polymers, amines, protonated amine oxides, betaines, and the like, which may act as antistatic agents in specific formulations and under controlled pH conditions in addition to any surfactant properties imparted by such materials. In addition to antistatic agents previously discussed, non-limiting examples of quaternary ammonium compounds useful as antistatic agents are acetamidopropyl trimonium chloride, behenamidopropyl dimethylamine, behenamidopropyl ethyldimonium ethosulfate, behentrimonium chloride, cetethyl morpholinium ethosulfate, cetrimonium chloride, cocoamidopropyl ethyldimonium ethosulfate, dicetyldimonium chloride, dimethicone hydroxypropyl trimonium chloride, hydroxyethyl behenamidopropyl dimonium chloride, quaternium-26, quaternium-27, quaternium-53, quaternium-63, quaternium-70, quaternium-72, quaternium-76 hydrolyzed collagen, PPG-9 diethylmonium chloride, PPG-25 diethylmonium chloride, PPG-40 diethylmonium chloride, stearalkonium chloride, stearamidopropyl ethyl dimonium ethosulfate, steardimonium hydroxypropyl hydrolyzed wheat protein, steardimonium hydroxypropyl hydrolyzed collagen, wheat germamidopropalkonium chloride, wheat germamidopropyl ethyldimonium ethosulfate, and the like.

Synthetic quaternary ammonium polymers include film-forming polymers and conditioning polymers. Non-limiting examples of synthetic quaternary ammonium polymers include polymers and copolymers of dimethyl diallyl ammonium chloride, such as polyquaternium-4, polyquaternium-6, polyquaternium-7, polyquaternium-22, polyquaternium-10, polyquaternium-11 polyquaternium-15, polyquaternium-16, polyquaternium-24, polyquaternium-28, polyquaternium-32, polyquaternium-33, polyquaternium-35, polyquaternium-37, polyquaternium-39, polyquaternium-43, polyquaternium-44, PEG-2-cocomonium chloride, and cassia hydroxypropyltrimonium chloride, quaternium-52, polyquaternium-46, polyquaternium-55, polyquaternium-44, polyquaternium-60, polyquaternium-66, polyquaternium-67, polyquaternium-68, polyquaternium-69, polyquaternium-72, polyquaternium-77, polyquaternium-85, polyquaternium-86, polyquaternium-87, and the like.

Suitable commercial conditioning polymers include polymeric quaternary ammonium salts such as, without being limited thereto, polyquaternium-7, a polymeric quaternary ammonium salt of acrylamide and dimethyl diallylammonium chloride monomers (such as MACKERNIUM^{™}-007, McIntyre Group, Ltd.); polyquaternium-10, a polymeric quaternary ammonium salt of hydroxyethylcellulose reacted with a trimethylammonium substituted epoxide (such as the UCARE^{®} Polymers JR, LK, LR, SR series, Amerchol and CELQUAT^{®} SC series, National Starch); polyquaternium-39, a polymeric quaternary ammonium salt of acrylic acid, diallyl dimethylammonium chloride and acrylamide (such as the MERQUAT^{®} and MERQUAT^{®} Plus polymers, Ondeo Nalco); quaternized derivatives of natural gums, e.g., guar hydroxypropyltrimonium chloride (such as the JAGUAR^{®} and JAGUAR^{®} Excel polymers, Rhodia, Inc.), and the like.

A number of non-polymeric quaternary ammonium compounds are used for fabric conditioning and fabric care, generally referred to as fabric softening agents, and are typically employed in amounts of up to about 20 weight percent of the total weight of the formulation, but are not limited thereto. Fabric softening agents useful in combination with the surfactant-polymer blend compositions of the present invention generally include long-chain alkylated quaternary ammonium compounds such as dialkyldimethyl quaternary ammonium compounds, imidazoline quaternary compounds, amidoamine quaternary compounds, dialkyl ester quat derivatives of dihydroxypropyl ammonium compounds; dialkyl ester quat derivatives of methyltriethanol ammonium compounds, ester amide amine compounds, and diester quat derivatives of dimethyldiethanol ammonium chloride, as described in the review article by Whalley, Fabric Conditioning Agents, HAPPI, pp. 55 to 58 (February 1995).

In addition to the previously discussed antistatic agents, non-limiting examples of dialkyldimethyl quaternary ammonium compounds include N,N-dioleyl-N,N-dimethylammonium chloride, N,N-ditallowyl-N,N-dimethylammonium ethosulfate, N,N-di(hydrogenated-tallowyl)-N,N-dimethylammonium chloride, and the like. Non-limiting examples of imidazoline quaternary compounds include 1-N-methyl-3-N-tallowamidoethylimidazolium chloride, 3-methyl-1-tallowylamidoethyl-2-tallowyl-imidazolinium methylsulfate, available from Witco Chemical Company under the tradename VARISOFT® 475, and the like. Non-limiting examples of amidoamine quaternary compounds include N-alkyl-N-methyl-N,N-bis(2-tallowamidoethyl)ammonium salts where the alkyl group can be methyl, ethyl, hydroxyethyl, and the like. Non-limiting examples of dialkyl ester quat derivatives of dihydroxypropyl ammonium compounds include 1,2-ditallowoyloxy-3-N,N,N-trimethylammoniopropane chloride, 1,2-dicanoloyloxy-3-N,N,N-trimethylammoniopropane chloride, and the like.

In addition, other types of long chain (e.g., natural oil and fatty acid-derived) alkylated quaternary ammonium compounds are suitable fabric softening agents, including, but not limited, to N,N-di(alkyloxyethyl)-N,N-dimethylammonium salts such as N,N-di(tallowyloxyethyl)-N,N-dimethylammonium chloride, N,N-di(canolyloxyethyl)-N,N-dimethylammonium chloride, and the like; N,N-di(alkyloxyethyl)-N-methyl-N-(2-hydroxyethyl)ammonium salts such as N,N-di(tallowyloxyethyl)-N-methyl-N-(2-hydroxyethyl)ammonium chloride, N,N-di(canolyloxyethyl)-N-methyl-N-(2-hydroxyethyl)ammonium chloride, and the like; N,N-di(2-alkyloxy-2-oxoethyl)-N,N-dimethylammonium salts, such as N,N-di(2-tallowyloxy-2-oxoethyl)-N,N-dimethylammonium chloride, N,N-di(2-canolyloxy-2-oxoethyl)-N,N-dimethylammonium chloride, and the like; N,N-di(2-alkyloxyethylcarbonyloxyethyl)-N,N-dimethylammonium salts, such as N,N-di(2-tallowyloxyethylcarbonyloxyethyl)-N,N-dimethylammonium chloride, N,N-di(2-canolyloxyethylcarbonyloxyethyl)-N,N-dimethylammonium chloride, and the like; N-(2-alkanoyloxy-2-ethyl)-N-(2-alkyloxy-2-oxoethyl)-N,N-dimethyl ammonium salts, such as N-(2-tallowoyloxy-2-ethyl)-N-(2-tallowyloxy-2-oxoethyl)-N,N-dimethyl ammonium chloride, N-(2-canoloyloxy-2-ethyl)-N-(2-canolyloxy-2-oxoethyl)-N,N-dimethyl ammonium chloride, and the like; N,N,N-tri(alkyloxyethyl)-N-methyl ammonium salts, such as N,N,N-tri(tallowyloxyethyl)-N-methylammonium chloride, N,N,N-tri(canolyloxyethyl)-N-methylammonium chloride, and the like; N-(2-alkyloxy-2-oxoethyl)-N-alkyl-N,N-dimethyl ammonium salts, such as N-(2-tallowyloxy-2-oxoethyl)-N-tallowyl-N,N-dimethyl ammonium chloride, N-(2-canolyloxy-2-oxoethyl)-N-canolyl-N,N-dimethyl ammonium chloride, and the like.

In one embodiment, the long-chain alkyl groups are derived from tallow, canola oil, or from palm oil, however, other alkyl groups derived from soybean oil and coconut oil, for example, are also suitable, as are lauryl, oleyl, ricinoleyl, stearyl, palmityl, and like fatty alkyl groups. The quaternary ammonium salt compounds can have any anionic group as a counter-ion, for example, chloride, bromide, methosulfate (i.e., methylsulfate), acetate, formate, sulfate, nitrate, and the like.

Examples of suitable quaternary ammonium fabric softening compounds include N-methyl-N,N-bis(tallowamidoethyl)-N-(2-hydroxyethyl)ammonium methylsulfate and N-methyl-N,N-bis(hydrogenated-tallowamidoethyl)-N-(2-hydroxyethyl) ammonium methylsulfate, each of which materials are available from Witco Chemical Company under the trade names VARISOFT^{®} 222 and VARISOFT^{®} 110, respectively; dialkyl esterquat derivatives of methyltriethanol ammonium salts such as the DEHYQUART^{®} AU series of bis(acyloxyethyl) hydroxyethylmethylammonium methosulfate esterquats available from Cognis, such as DEHYQUART^{®} AU35, AU46, AU56, and the like; and N,N-di(tallowoyloxyethyl)-N,N-dimethylammonium chloride, where the tallow chains are at least partially unsaturated. Other suitable fabric softening agents include the well-known dialkyldimethyl ammonium salts such as N,N-ditallowyl-N,N-dimethyl ammonium methylsulfate, N,N-di(hydrogenated-tallowyl)-N,N-dimethyl ammonium chloride, N,N-distearyl-N,N-dimethyl ammonium chloride, N,N-dibehenyl-N,N-dimethylammonium chloride, N,N-di(hydrogenated tallow)-N,N-dimethyl ammonium chloride (trade name ADOGEN^{®} 442), N,N-ditallowyl-N,N-dimethyl ammonium chloride (trade name ADOGEN^{®} 470, PRAEPAGEN^{®} 3445), N,N-distearyl-N,N-dimethyl ammonium chloride (trade name AROSURF^{®} TA-100), all available from Witco Chemical Company; N,N-dibehenyl-N,N-dimethyl ammonium chloride, sold under the trade name KE-MAMINE° Q-2802C by Humko Chemical Division of Witco Chemical Corporation; and N,N-dimethyl-N-stearyl-N-benzylammonium chloride sold under the trade names VARISOFT® SDC by Witco Chemical Company and AMMONYX^{®} 490 by Onyx Chemical Company.

Any of the foregoing fabric softening agents, and mixtures thereof, can be utilized in combination with the surfactant-polymer blend compositions of the present invention, particularly in laundry and fabric care products. For ester-containing fabric softening agents, the pH of the compositions can influence the stability of the fabric softening agents, especially in prolonged storage conditions. The pH, as defined in the present context, is measured in the neat compositions at 20°C. In one embodiment, the pH of the composition is less than 6. For optimum hydrolytic stability of these compositions, the pH is in the range of from 2 to 5, or from 2.5 to 3.5.

In addition to protein derivatives previously described, non-limiting examples of protein derivatives include cocodimonium hydroxypropyl hydrolyzed casein, cocodimonium hydroxypropyl hydrolyzed collagen, cocodimonium hydroxypropyl hydrolyzed hair keratin, cocodimonium hydroxypropyl hydrolyzed rice protein, cocodimonium hydroxypropyl hydrolyzed silk, cocodimonium hydroxypropyl hydrolyzed soy protein, cocodimonium hydroxypropyl hydrolyzed wheat protein, cocodimonium hydroxypropyl hydrolyzed silk amino acids, hydroxypropyl trimonium hydrolyzed collagen, hydroxypropyl trimonium hydrolyzed keratin, hydroxypropyl trimonium hydrolyzed silk, hydroxypropyl trimonium hydrolyzed rice bran, hydroxypropyl trimonium hydrolyzed soy protein, hydroxypropyl trimonium hydrolyzed vegetable protein, hydroxypropyl trimonium hydrolyzed wheat protein, soyethyldimonium ethosulfate, soyethyl morpholinium ethosulfate, and the like.

### Institutional and Industrial Applications

The surfactant-polymer blends of the present invention can be utilized as thickeners, rheology modifiers, dispersants, stabilizers, promoters, and the like, in institutional and industrial product applications, such as, without being limited thereto, textiles (e.g., processing, finishing, printing, and dyeing aids, protective washable surface coatings, manufacture of synthetic leather by saturation of non-woven fabrics, and the like, manufacturing of woven fabrics, non-woven fabrics, natural and synthetic fibers and the like); water treatments (e.g., waste water, cooling water, potable water purification, and the like); chemical spill containments (e.g., acid-spill absorbent, and the like); leather and hide processing (e.g., processing aids, finishing, coating, embossing, and the like); paper and papermaking (e.g., surface coatings, such as pigmented coatings, antistatic coatings, and the like, pulp binders, surface sizings, dry and wet strength enhancers, manufacture of wet-laid felts, and the like); printing (e.g., inks, anti-wicking ink-jet printer inks, thickeners for ink formulations containing cationic dyes for printing acrylic fabrics, and the like); paints (e.g., pigment and grinding additive, crosslinking agent for epoxy latex emulsions, particulate-suspending aid for clays, pigments, and the like); industrial plant effluent treatment (e.g., flocculants for phenolics in paper mill effluent, and the like); metal working (e.g., acid etch cleaners, low pH metal coatings, pickling agents in cold rolled steel processing, and the like); adhesives (e.g., clear adhesives, adhesion promoters for metal, plastic, wood, and the like, non-woven floc adhesive tie coatings, bonding, and the like); wood preservation; and industrial construction products for buildings and roads (e.g., cement plasticizers, asphalt emulsion stabilizers at low pH, acid etch for cement, consistency modifiers of concrete, mortar, putty, and the like). The surfactant-polymer blends of the present invention are particularly useful as thickeners for rust removers, acid truck cleaners, scale removers, and the like.

In one embodiment, the surfactant-polymer blend is added to a desired personal care, health care, home care, and "|&|" formulation and the pH is adjusted upward with an alkaline material such as an organic base to optimize swelling of the polymer in the blend to the desired viscosity, and then adjusting the final composition to the desired pH. If the pH of a completed composition or formulation containing the surfactant-polymer blend is more alkaline than required for the intended use of the formulation, the pH can then be further adjusted with any, physiologically tolerable, inorganic or organic acid.

The surfactant-polymer blends of the invention are useful as a thickener for antistatic, biocidal, antimicrobial, and other preservative compositions, in a variety of personal care, health care, |&|, and medical applications. For example, the polymer can be employed as a thickener in over-the-counter (OTC) health care and pharmaceutical products where cationic biocides are typically employed, such as in oral care compositions for plaque and tartar control, and liquid vehicles containing therapeutic agents, such as syrups, gels, and the like.

Products containing a surfactant-polymer blend of the invention can be packaged and dispensed from containers, such as jars, bottles, tubes, spray bottles, wipes, cans, roll-on containers, stick containers, and the like, without limitation. There is no limitation as to the form of product in which the surfactant-polymer can be incorporated, so long as the purpose for which the product is used is achieved. For example, personal care and health care products containing a surfactant-polymer can be applied to the skin, hair, scalp and nails in the form of, without being limited thereto, gels, mousses, sprays (liquid or foam), emulsions (creams, lotions, pastes), liquids (rinses, shampoos), bars, ointments, suppositories, impregnated wipes, patches, and the like.

### Additives and Adjuvants

Product formulations comprising the surfactant-polymer blends of this invention can contain the various additives and adjuvants previously described and/or conventionally or popularly included in personal care, health care, home care, "|&|" care products, and in industrial processes, including, without being limited thereto, acidifying or alkalizing pH adjusting agents and buffering agents; auxiliary fixatives and film formers (e.g., nonionic, anionic, cationic, or amphoteric polymers of synthetic or natural origin); auxiliary rheology modifiers (e.g., viscosity-increasing polymeric, gum, or resin thickeners or gellants); additives (e.g., auxiliary, auxiliary emulsifiers, auxiliary emulsion stabilizers, waxes, auxiliary dispersants, and the like), viscosity control agents (e.g., electrolytes), auxiliary conditioning agents (e.g., synthetic oils, natural oils, animal oils, natural and synthetic waxes, silicones, monomeric and polymeric quaternized ammonium compounds (previously described) and derivatives thereof), sheen enhancers; moisturizers; emollients; humectants; lubricants; sunscreen agents; UV absorbing agents; oxidizing agents; reducing agents; surfactants (e.g., anionic, cationic, nonionic, amphoteric, zwitterionic, and silicone derivatives thereof, and mixtures thereof); polymer film modifying agents (e.g., plasticizers, tackifiers, de-tackifiers, wetting agents, and the like); chelating agents; opacifiers; pearlescing agents; proteinaceous materials and derivatives thereof; vitamins and derivatives thereof; botanicals; antifungal agents; antidandruff agents; anti-inflammatory agents; analgesics; preservatives; fragrances; fragrance solubilizers; colorants (e.g., pigments and dyes); propellants (e.g., fluorinated hydrocarbons, liquid volatile hydrocarbons, compressed gases, and the like; and mixtures thereof.

Additives and adjuvant ingredients, products, or materials, which may be employed with the inventive surfactant-polymer blend compositions discussed herein are in some cases referred to by the international nomenclature commonly referred to as INCI name given them in the International Cosmetic Ingredient Dictionary, published by the Cosmetic, Toiletry, and Fragrance Association, Washington D.C. (see www.ctfa-online.org - hereafter INCI Dictionary), such as can be found in any edition thereof, for example, Volumes 1 and 2, Sixth Edition, (1995) or Volumes 1-3, Seventh and Eighth Editions, (1997, 2000), or by their commonly used chemical names. Numerous commercial suppliers of materials listed by INCI name, trade name or both can be found in the INCI Dictionary and in numerous commercial trade publications, including but not limited to the 2001 McCutcheon's Directories, Volume 1: Emulsifiers & Detergents and Volume 2: Functional Materials, published by McCutcheon's Division, The Manufacturing Confectioner Publishing Co., Glen Rock, N.J. (2001); and 2001 Cosmetic Bench Reference, edition of Cosmetics & Toiletries® 115 (13), published by Allured Publishing Corporation, Carol Stream, III. (2001). Such components and the formulation of compositions are also described in detail in well known references, such as Cosmetics Science and Technology, First Edition (Sagarin (ed)), published 1957, and Second Edition (Balsam, et al. (eds)), published 1972 to 1974; and The Chemistry and Manufacture of Cosmetics, Second Edition (deNavarre (ed)), published 1975, and Third Edition (Schlossman (ed)), published 2000, both available from Allured Publishing Corporation; Rieger (ed), Harry's Cosmeticology, 8th Edition, Chemical Publishing, Co., Inc., New York, N.Y. (2000); and various formularies available to those skilled in the pharmaceutical arts, such as Remington's Pharmaceutical Sciences, Fourteenth Edition, Mack Publishing Company, Easton, Pa. (1970).

It is known that formulated compositions for personal care and topical, dermatological, health care, which are applied to the skin and mucous membranes for cleansing or soothing, are compounded with many of the same or similar physiologically tolerable ingredients and formulated in the same or similar product forms, differing primarily in the purity grade of ingredient selected, by the presence of medicaments or pharmaceutically accepted compounds, and by the controlled conditions under which products may be manufactured. Likewise, many of the ingredients employed in products for home care and |&| are the same or similar to the foregoing, differing primarily in the amounts and material grade employed. It is also known that the selection and permitted amount of ingredients also may be subject to governmental regulations, on a national, regional, local, and international level. Thus, discussion herein of various useful ingredients for personal care and health care products may apply to home care and |&| products and industrial applications.

### Solvents

The polymers of the present invention prepared as aqueous emulsions are particularly useful for water-based formulations, and formulations containing water-miscible auxiliary solvents, but are not limited thereto. Useful solvents commonly employed are typically liquids, such as water (deionized, distilled or purified), alcohols, polyols, and the like, and mixtures thereof. Non-aqueous or hydrophobic auxiliary solvents are commonly employed in substantially water-free products, such as nail lacquers, aerosol propellant sprays, or for specific functions, such as removal of oily soils, sebum, make-up, or for dissolving dyes, fragrances, and the like, or are incorporated in the oily phase of an emulsion. Non-limiting examples of auxiliary solvents, other than water, include linear and branched alcohols, such as ethanol, propanol, isopropanol, hexanol, and the like; aromatic alcohols, such as benzyl alcohol, cyclohexanol, and the like; saturated C₁₂ to C₃₀ fatty alcohol, such as lauryl alcohol, myristyl alcohol, cetyl alcohol, stearyl alcohol, behenyl alcohol, and the like. Non-limiting examples of polyols include polyhydroxy alcohols, such as glycerin, propylene glycol, butylene glycol, hexylene glycol, C₂ to C₄ alkoxylated alcohols and C₂ to C₄ alkoxylated polyols, such as ethoxylated, propoxylated, and butoxylated ethers of alcohols, diols, and polyols having 2 to 30 carbon atoms and 1 to 40 alkoxy units, polypropylene glycol, polybutylene glycol, and the like. Non-limiting examples of non-aqueous auxiliary solvents include silicones, and silicone derivatives, such as cyclomethicone, and the like, ketones such as acetone and methylethyl ketone; natural and synthetic oils and waxes, such as vegetable oils, plant oils, animal oils, essential oils, mineral oils, C₇ to C₄₀ isoparaffins, alkyl carboxylic esters, such as ethyl acetate, amyl acetate, ethyl lactate, and the like, jojoba oil, shark liver oil, and the like. Some of the foregoing non-aqueous auxiliary solvents may also be conditioners and emulsifiers.

### Propellants

Suitable propellants that can be utilized in compositions comprising the surfactant-polymer blend compositions of the invention include but are not limited to Where applicable, any known aerosol propellant can be utilized to deliver the personal care, home care, health care and institutional' care compositions containing the esters of the present invention in combination with one or more of the foregoing active ingredients and/or with the one or more additives and/or adjuvants, conventionally or popularly included in personal care, health care, home care, and institutional care products discussed above. Exemplary propellants include, but are not limited to, lower boiling hydrocarbons such as C₃ to C₆ straight and branched chain hydrocarbons. Exemplary hydrocarbon propellants include propane, butane, isobutene, and mixtures thereof. Other suitable propellants include ethers, such as, dimethyl ether, hydrofluorocarbons, such as, 1,1-difluoroethane, and compressed gasses, such as air and carbon dioxide. These compositions can contain from 0.5 weight percent to 60 weight percent of the propellant in one embodiment and from 0.5 weight percent to 35 weight percent in another embodiment, based on the total weight of the composition.

### pH Adjusting Agents

Suitable acidic pH adjusting agents are selected from organic acids, including amino acids, and inorganic mineral acids. Examples of acidic pH adjusting agents include, but are not limited to, acetic acid, salicylic acid, citric acid, fumaric acid, glutamic acid, glycolic acid, hydrochloric acid, lactic acid, nitric acid, phosphoric acid, sodium bisulfate, sulfuric acid, tartaric acid, and the like, and mixtures thereof. Suitable alkaline or basic pH adjusting agents can be added to swell the polymer contained in the surfactant-polymer blend of the invention. Examples of alkaline pH adjusting agents include, but are not limited to, alkali metal hydroxides, such as sodium hydroxide, and potassium hydroxide; ammonium hydroxide; organic bases, such as triethanolamine, diisopropylamine, dodecylamine, diisopropanolamine, aminomethyl propanol, cocamine, oleamine, morpholine, triamylamine, triethylamine, tromethamine (2-amino-2-hydroxymethyl)-1,3-propanediol), and tetrakis(hydroxypropyl)ethylene-diamine; and alkali metal salts of inorganic acids, such as sodium borate (borax), sodium phosphate, sodium pyrophosphate, and the like, and mixtures thereof. The acidic and alkaline pH adjusting agent can be utilized in any amount necessary to obtain a desired pH value in the final composition.

### Silicones

Silicones are commonly used in shampoo products, such as the so-called "two-in-one" combination cleansing/conditioning shampoos, and in skin care products as well. Silicones provide conditioning properties as well as psychosensory and aesthetic properties to a formulation in which they are included, making the hair and skin feel softer and smoother to the touch. Silicones can also functions as emulsifiers and as emollients in a personal care formulation. The most common class of silicone polymers are the linear polydimethyl siloxanes having the general formula (CH₃)₃-Si(CH₃)₂-O-(Si(CH₃)₂-O)_{w}-Si(CH₃)₃ where w is an integer greater than 2. Silicones can also be branched materials wherein one or more alkyl groups in a polymer are replaced with oxygen to create a branch point. The silicon atoms may carry a wide variety of substituents which can be the same or different. The chemically least reactive substituents are the methyl or phenyl groups. Functional endblocking groups may carry nitrogen or hydroxyl moieties, as in the case of dimethiconol. Background material on silicones including sections discussing silicone fluids, gums, and resins, as well as manufacture of silicones, are found in Encyclopedia of Polymer Science and Engineering, Vol. 15, 2d ed., pp. 204 to 308, John Wiley & Sons, Inc. (1989).

Silicone fluids are typically water-insoluble oils having a viscosity in the range of a few mPa·s to several hundred thousand mPa·s. They are in the form of fluids (volatile and non-volatile), gums, gums in fluids, resins or in the form of nonionic small and large particle size emulsions (MEM 1664, MEM 1310, 5-7137, 2-1352, MEM 1784, MEM 1310, MEM 1491, 5-7137, and MEM 2220 from Dow Corning), and anionic emulsions (MEM 1784 from Dow Corning). The refractive index of the polysiloxane fluid will generally be less than 1.70, typically less than 1.60. In this context, polysiloxane "fluid" includes oils as well as gums.

Another class of silicones for use in hair care products are the so-called rigid silicones (also known as silicone gums), as described, for example in United States Patent No. 4,902,499, which generally have a viscosity (at 20°C) of greater than 600,000 mPa·s and have a weight average molecular weight of at least 500,000 Daltons as determined by intrinsic viscosity measurement. Gums are also described in United States Patent No. 4,152,416; Noll and Walter, Chemistry and Technology of Silicones, New York: Academic Press (1968); and in General Electric Silicone Rubber Product Data Sheets SE 30, SE 33, SE 54 and SE 76. Specific non-limiting examples of gums for use in the compositions of the present invention include polydimethylsiloxane, (polydimethylsiloxane) (methylvinylsiloxane) copolymer, poly(dimethylsiloxane) (diphenyl siloxane)(methylvinylsiloxane) copolymer and mixtures thereof.

Volatile silicones are particularly useful in combination with the polymers of the present invention and are often used as lubricants in hair care products, such as shampoos. Volatile silicones include cyclic and linear polydimethylsiloxanes, and the like. Cyclic volatile silicones typically contain 3 to 7 silicon atoms, alternating with oxygen atoms, in a cyclic ring structure. Each silicon atom is also substituted with two alkyl groups, typically methyl groups. Linear volatile silicones are silicone fluids, as described above, having viscosities of not more than 25 mPa·s. A description of volatile silicones is found in Todd and Byers, Volatile Silicone Fluids for Cosmetics, Cosmetics and Toiletries, Vol. 91(1), pp. 27 to 32 (1976), and in Kasprzak, Volatile Silicones, Soap/Cosmetics/Chemical Specialties, pp. 40 to 43 (December 1986).

Another class of silicones includes aminosilicone category which contains any amine functionalized silicone; i.e., a silicone containing at least one primary amine, secondary amine, tertiary amine, or a quaternary ammonium group. Aminosilicones can be graft or terminal. In one embodiment, the aminosilicone has a viscosity of from 1,000.10⁻⁶ m²/s (cs) to 1,000,000.10⁻⁶ m²/s (cs), or from 2,000·10⁻⁶ m²/s (cs) to 600,000·10⁻⁶ m²/s (cs), or even from 4,000·10⁻⁶ m²/s (cs) to 400,000·10⁻⁶ m²/s (cs). Examples of aminosilicone fluids with low and high amine content (8500, 2-8566, AP-8087 from Dow Corning); amino glycol copolymer; amino phenyl resin; low and high viscosity cationic emulsions (949, 2-8194 from Dow Corning), nonionic microemulsions; silicone quat microemulsions (5-7113 from Dow Corning), and mixtures of any two or more thereof.

Other silicone oils include the dimethicone copolyols, which are linear or branched copolymers of dimethylsiloxane (dimethicone) and alkylene oxides. The dimethicone polyols can be random or block copolymers. A generally useful class of dimethicone polyols are block copolymers having blocks of polydimethylsiloxane and blocks of polyalkylene oxide, such as blocks of polyethylene oxide, polypropylene oxide, or both. Dimethicone copolyols are disclosed in United States Patent Nos. 5,136,063 and 5,180,843. In addition, dimethicone copolyols are commercially available under the Silsoft^{®} and Silwet^{®} brand names from the General Electric Company (GE-OSi). Specific product designations include but are not limited to Silsoft 305, 430, 475, 810, 895, Silwet L 7604 (GE-OSi); Dow Corning^{®} 5103 and 5329 from Dow Corning Corporation; and Abil^{®} dimethicone copolyols, such as, for example WE 09, WS 08, EM 90 and EM 97 from Evonik Goldschmidt Corporation; and Silsense^{™} dimethicone copolyols, such as Silsense Copolyol-1 and Silsense Copolyol-7, available from Lubrizol Advanced Materials, Inc.

Silicone materials, including volatile silicones, silicone gums, and silicone copolymers, mixtures of dimethicones and dimethiconols; phenyl-modified silicones, alkyl/alkoxy-modified silicones, polyether functional silicones, polyglycerin-modified silicones, polyether/alkyl-modified silicones, polyglycerin/alkyl-modified silicones, silicone cross-polymers, silicone resin, silicone resin gels, silicone polyglucosides, are available from a variety of commercial sources such as Dow Corning, Shin-Etsu, Wacker, General Electric Company, Momentive Performance Materials, and Lubrizol.

The silicones used in the present invention have a particle size of from 0.1 µm to 300 µm or from 5 µm to 80 µm, or from 20 µm to 60 µm, or even from 30 µm to 50 µm.

### Conditioners

In addition to the silicone and quaternary (monomeric and polymeric) conditioners previously disclosed, exemplary conditioners include synthetic oil conditioners selected from polyolefins, e.g., poly-α-olefins such as polybutenes, polyisobutenes and polydecenes. The polyolefins can be hydrogenated. Fluorinated or perfluorinated oils are also contemplated within the scope of the present invention. Fluorinated oils include perfluoropolyethers described in European Patent 0 486 135 and the fluorohydrocarbon compounds described in WO 93/11103. The fluoridated oils may also be fluorocarbons such as fluoramines, e.g., perfluorotributylamine, fluoridated hydrocarbons, such as perfluorodecahydronaphthalene, fluoroesters, and fluoroethers.

Suitable natural oil conditioners include but are not limited to peanut, sesame, avocado, coconut, cocoa butter, almond, safflower, corn, cotton seed, sesame seed, walnut oil, castor, olive, jojoba, palm, palm kernel, soybean, wheat germ, linseed, sunflower seed; eucalyptus, lavender, vetiver, litsea, cubeba, lemon, sandalwood, rosemary, chamomile, savory, nutmeg, cinnamon, hyssop, caraway, orange, geranium, cade, and bergamot oils, fish oils, glycerol tricaprocaprylate; and mixtures thereof.

Suitable natural and synthetic wax conditioning agents include but are not limited to carnauba wax, candelila wax, alfa wax, paraffin wax, ozokerite wax, olive wax, rice wax, hydrogenated jojoba wax, bees wax, modified bees wax, e.g., cerabellina wax, marine waxes, polyolefin waxes, e.g., polyethylene wax; and mixtures thereof.

In one aspect, the conditioning agent(s) can be present in an amount of 0.001 weight percent to 20 weight percent in one embodiment, from 0.01 weight percent to 10 weight percent in another embodiment, and from 0.1 weight percent to 3 weight percent in still yet another embodiment, based on the total weight of the composition.

### Emulsifiers

Exemplary emulsifiers include but are not limited to C₁₂ to C₁₈ fatty alcohols; alkoxylated C₁₂ to C₁₈ fatty alcohols; C₁₂ to C₁₈ fatty acids; and alkoxylated C₁₂ to C₁₈ fatty acids, the alkoxylates each having 10 to 30 units of ethylene oxide, propylene oxide, and combinations of ethylene oxide/propylene oxide; C₈ to C₂₂ alkyl mono- and oligoglycosides; ethoxylated sterols; partial esters of polyglycerols; esters and partial esters of polyols having 2 to 6 carbon atoms and saturated and unsaturated fatty acids having 12 to 30 carbon atoms; partial esters of polyglycerols; and organosiloxanes; and combinations thereof.

The fatty alcohols, acids and alkoxylated fatty alcohols and fatty acids are as described in the emollient description above. In one aspect of the invention, the fatty alcohols and fatty acids each are ethoxylated with 10 to 30 units of ethylene oxide.

The C₈ to C₂₂ alkyl mono- and oligoglycoside emulsifiers are prepared by reacting glucose or an oligosaccharide with primary fatty alcohols having 8 to 22 carbon atoms. Products which are obtainable under the trademark Plantacare^{®} comprise a glucosidically bonded C₈ to C₁₆ alkyl group on an oligoglucoside residue whose average degree of oligomerization is 1 to 2. Exemplary alkyl glucosides and oligoglycosides are selected from octyl glucoside, decyl glucoside, lauryl glucoside, palmityl glucoside, isostearyl glucoside, stearyl glucoside, arachidyl glucoside and behenyl glucoside, and mixtures thereof. These glucosides can also function as nonionic surfactants.

Exemplary ethoxylated sterols include ethoxylated vegetable oil sterols such as, for example, soya sterols. The degree of ethoxylation is greater than 5 in one aspect, and at least 10 in another aspect. Suitable ethoxylated sterols are PEG-10 Soy Sterol, PEG-16 Soy Sterol and PEG-25 Soy Sterol.

The partial esters of polyglycerols have 2 to 10 glycerol units and are esterified with 1 to 4 saturated or unsaturated, linear or branched, optionally hydroxylated C₈ to C₃₀ fatty acid residues. Representative partial esters of polyglycerols include diglycerol monocaprylate, diglycerol monocaprate, diglycerol monolaurate, triglycerol monocaprylate, triglycerol monocaprate, triglycerol monolaurate, tetraglycerol monocaprylate, tetraglycerol monocaprate, tetraglycerol monolaurate, pentaglycerol monocaprylate, pentaglycerol monocaprate, pentaglycerol monolaurate, hexaglycerol monocaprylate, hexaglycerol monocaprate, hexaglycerol monolaurate, hexaglycerol monomyristate, hexaglycerol monostearate, decaglycerol monocaprylate, decaglycerol monocaprate, decaglycerol monolaurate, decaglycerol monomyristate, decaglycerol monoisostearate, decaglycerol monostearate, decaglycerol monooleate, decaglycerol monohydroxystearate, decaglycerol dicaprylate, decaglycerol dicaprate, decaglycerol dilaurate, decaglycerol dimyristate, decaglycerol diisostearate, decaglycerol distearate, decaglycerol dioleate, decaglycerol dihydroxystearate, decaglycerol tricaprylate, decaglycerol tricaprate, decaglycerol trilaurate, decaglycerol trimyristate, decaglycerol triisostearate, decaglycerol tristearate, decaglycerol trioleate, decaglycerol trihydroxystearate, and mixtures thereof.

The saturated C₁₂ to C₃₀ fatty alcohol emulsifiers are as described in the emollient description set forth above. In one aspect of the invention, the fatty alcohol emulsifier is selected from but not limited to cetyl alcohol, stearyl alcohol, arachidyl alcohol, behenyl alcohol and lanolin alcohol or mixtures of these alcohols, and as are obtainable in the hydrogenation of unsaturated vegetable oil and animal fatty acids.

Emulsifiers based on the esters and partial esters of polyols having 2 to 6 carbon atoms and linear saturated and unsaturated fatty acids having 12 to 30 carbon atoms are, for example, the monoesters and diesters of glycerol or ethylene glycol or the monoesters of propylene glycol with saturated and unsaturated C₁₂ to C₃₀ fatty acids.

The partially esterified polyglycerol emulsifiers include 2 to 10 glycerol units and esterified with 1 to 5 saturated or unsaturated, linear or branched, optionally hydroxylated C₈ to C₃₀ fatty acid residues.

The organosiloxane emulsifiers are polymeric emulsifiers that contain at least one hydrophobic portion and at least one hydrophilic portion. The polymer backbone contains repeating siloxy units that can have cyclic, linear or branched repeating units, e.g. di(C₁ to C₅)alkylsiloxy units, typically dimethylsiloxy units.

The hydrophilic portion of the organosiloxane is generally achieved by substitution onto the polymeric backbone of a residue that confers hydrophilic properties to a portion of the molecule. The hydrophilic residue may be substituted on a terminus of the polymeric organosiloxane, or on any one or more repeating units of the polymer. Generally, the hydrophilic residue is derived from ethylene oxide units that are grafted onto the polymer backbone. In general, the repeating dimethylsiloxy units of modified polydimethylsiloxane emulsifiers are hydrophobic in nature due to the methyl groups, and confer the hydrophobicity properties to the molecule. In addition, longer chain alkyl residues, hydroxy terminated polypropyleneoxy residues, hydroxy terminated polyether residues comprising a combination of ethylene oxide and propylene oxide residues, and/or other types of residues can be substituted onto the siloxy backbone to confer additional emulsification properties to the backbone. Polyether substituted organosiloxane emulsifiers are known as dimethicone copolyols and are widely commercially available. The dimethicone polyols can be random or block copolymers. A generally useful class of dimethicone polyols is block copolymers having blocks of polydimethylsiloxane and blocks of polyalkylene oxide, such as blocks of polyethylene oxide, polypropylene oxide, or both.

### Emollients

Suitable emollients include but are not limited to an emollient selected from silicone fluids (e.g., volatile silicone oils and non-volatile silicone oils); mineral oils; petrolatums; vegetable oils; fish oils; fatty alcohols; fatty acids; fatty acid and fatty alcohol esters; alkoxylated fatty alcohols; alkoxylated fatty acid esters; benzoate esters; panthenol; Guerbet esters; alkyl ether derivatives of polyethylene glycols, such as, for example methoxypolyethylene glycol (MPEG); and polyalkylene glycols; lanolin and lanolin derivatives; and the like. The emollient can be used alone or in combination with one or more emollients of the present invention.

Volatile silicone oils include cyclic and linear polydimethylsiloxanes, low molecular weight organo-functional silicones, and the like. Cyclic volatile silicones (cyclomethicones) typically contain 3 to 7 silicon atoms, alternating with oxygen atoms, in a cyclic ring structure. Each silicon atom is typically substituted with two alkyl groups, such as, for example, methyl groups. Volatile linear polydimethylsiloxanes (dimethicones) typically contain 2 to 9 silicon atoms, alternating with oxygen atoms in a linear arrangement. Each silicon atom is also substituted with two alkyl groups (the terminal silicon atoms are substituted with three alkyl groups), such as, for example, methyl groups. The linear volatile silicones typically have viscosities of less than about 5 mPa·s (cP) at 25°C., while the cyclic volatile silicones typically have viscosities of less than about 10 mPa·s (cP) at 25°C. "Volatile" means that the silicone has a measurable vapor pressure, or a vapor pressure of at least 267 Pa (2 mm of Hg) at 20°C. Non-volatile silicones have a vapor pressure of less than 267 Pa (2 mm Hg) at 20°C. A description of volatile silicones is found in Todd and Byers, "Volatile Silicone Fluids for Cosmetic Formulations," Cosmetics and Toiletries, Vol. 91, pp. 29 to 32 (January 1976), and in Kasprzak, "Volatile Silicones," Soap/Cosmetics/Chemical Specialties, pp. 40 to 43 (December 1986).

Exemplary volatile cyclomethicones are D4 cyclomethicone (octamethylcyclotetrasiloxane), D5 cyclomethicone (decamethylcyclopentasiloxane), D6 cyclomethicone, and blends thereof (e.g., D4/D5 and D5/D6). Volatile cyclomethicones and cyclomethicone blends are commercially available from G.E. Silicones as SF1173, SF1202, SF1256, and SF1258, Dow Corning Corporation as Dow Corning® 244, 245, 246, 345, and 1401 Fluids. Blends of volatile cyclomethicones and volatile linear dimethicones, are also contemplated.

Exemplary volatile linear dimethicones include hexamethyldisiloxane, octamethyltrisiloxane, decamethyltetrasiloxane, dodecamethylpentasiloxane and blends thereof. Volatile linear dimethicones and dimethicone blends are commercially available from Dow Corning Corporation as Dow Corning 200® Fluid (e.g., product designations 0.65 CST, 1°CST, 1.5 CST, and 2 CST) and Dow Corning^{®} 2-1184 Fluid.

Exemplary volatile low molecular weight organo-functional silicones include phenyl trimethicone, caprylyl trimethicone, caprylyl methicone, and hexyl methicone, and blends thereof. Low molecular weight organo-functional silicones are commercially available from Clariant under the trade name Silcare^{®} 41M10, Silcare^{®}31M60, Silcare^{®} 41M10, and Silcare^{®} 41M15.

The non-volatile silicone oils useful as emollients in the present invention are linear and typically have viscosities of from 10 mPa·s (cP) to 100,000 mPa·s(cP) at 25°C. They typically contain above 10 dialkyl/diaryl or monoalkyl/monoaryl substituted silicon atoms, alternating with oxygen atoms in a linear arrangement. They include polyalkylsiloxane, polyarylsiloxane, and polyalkylarylsiloxane polymers. Exemplary non-volatile silicone oils include the polydimethylsiloxanes (dimethicones), polydiethylsiloxanes, polymethylphenylsiloxanes, and the like. In one aspect of the invention, the non-volatile silicone oil is selected from a non-volatile polydimethylsiloxane having a viscosity range from 10 mPa·s (cP) to 100,000 mPa·s (cP) at 25°C. Non-volatile dimethicones are commercially available from Dow Corning Corporation as Dow Corning 200^{®} Fluid (product designations 10 CST through 10,000 CST).

Mineral oils and petrolatums include cosmetic, USP and NF grades and are commercially available from Penreco under the Drakeol^{®} and Penreco^{®} trade names. Mineral oil includes hexadecane and paraffin oil.

Exemplary vegetable oils suitable an emollient component in the present invention include but are not limited to peanut oil, sesame oil, avocado oil, coconut oil, cocoa butter, almond oil, safflower oil, corn oil, cotton seed oil, sesame seed oil, walnut oil, castor oil, olive oil, jojoba oil, palm oil, palm kernel oil, soybean oil, wheat germ oil, linseed oil, sunflower seed oil; and the mono-, di-, and triglycerides thereof. Exemplary mono-, di- and triglycerides are, for example, caprylic triglyceride, capric triglyceride, caprylic/capric triglyceride, and caprylic/capric/lauric triglyceride, caprylic/capric/stearic triglyceride, and caprylic/capric/linoleic triglyceride.

Ethoxylated mono- and diglycerides are also suitable as an emollient component of the present invention, such as, for example, PEG-8 Caprylic/Capric Glycerides.

Suitable fatty alcohol emollients include but are not limited to fatty alcohols containing 8 to 30 carbon atoms. Exemplary fatty alcohols include capryl alcohol, pelargonic alcohol, capric alcohol, lauryl alcohol, myristyl alcohol, cetyl alcohol, isocetyl alcohol, stearyl alcohol, isostearyl alcohol, cetearyl alcohol, oleyl alcohol, ricinoleyl alcohol, arachidyl alcohol, icocenyl alcohol, behenyl alcohol, and mixtures thereof.

Suitable fatty acid emollients include but are not limited to fatty acids containing 10 to 30 carbon atoms. Exemplary fatty acids are selected from capric acid, lauric acid, myristic acid, palmitic acid, stearic acid, oleic acid, linoleic acid, arachidic acid, behenic acid, and mixtures thereof.

Suitable fatty acid and fatty alcohol ester emollients include but are not limited to hexyl laurate, decyl oleate, isopropyl stearate, isopropyl isostearate, butyl stearate, octyl stearate, cetyl stearate, myristyl myristate, octyldodecyl stearoylstearate, octylhydroxystearate, diisopropyl adipate, isopropyl myristate, isopropyl palmitate, ethyl hexyl palmitate, isodecyl oleate, isodecyl neopentanoate, diisopropyl sebacate, isostearyl lactate, lauryl lactate, diethyl hexyl maleate, PPG-14 butyl ether and PPG-2 myristyl ether propionate, cetearyl octanoate, and mixtures thereof.

Alkoxylated fatty alcohols are ethers formed from the reaction of a fatty alcohol with an alkylene oxide, generally ethylene oxide or propylene oxide. Suitable ethoxylated fatty alcohols are adducts of fatty alcohols and polyethylene oxide. In one aspect of the invention, the ethoxylated fatty alcohols can be represented by the formula R-(OCH₂CH₂)ₙ-OH, wherein R represents the aliphatic residue of the parent fatty alcohol and n represents the number of molecules of ethylene oxide. In another aspect of the invention, R is derived from a fatty alcohol containing 8 to 30 carbon atoms. In one aspect, n is an integer ranging from 2 to 50, 3 to 25 in another aspect, and 3 to 10 in a further aspect. In a still further aspect, R is derived from a fatty alcohol emollient set forth above. Exemplary ethoxylated fatty alcohols are but are not limited to capryl alcohol ethoxylate, lauryl alcohol ethoxylate, myristyl alcohol ethoxylate, cetyl alcohol ethoxylate, stearyl alcohol ethoxylate, cetearyl alcohol ethoxylate oleyl alcohol ethoxylate, and, behenyl alcohol ethoxylate, wherein the number of ethylene oxide units in each of the foregoing ethoxylates can range from 2 and above in one aspect, and from 2 to 150 in another aspect. It is to be recognized that the propoxylated adducts of the foregoing fatty alcohols and mixed ethoxylated/propoxylated adducts of the foregoing fatty alcohols are also contemplated within the scope of the invention. The ethylene oxide and propylene oxide units of the ethoxylated/propoxylated fatty alcohols can be arranged in random or in blocky order.

More specific examples of ethoxylated alcohols are but are not limited to Beheneth 5-30 (the 5-30 meaning the range of repeating ethylene oxide units), Ceteareth 2-100, Ceteth 1-45, Cetoleth 24-25, Choleth 10-24, Coceth 3-10, C9-11 pareth 3-8, C11-15 pareth 5-40, C11-21 Pareth 3-10, C12-13 pareth 3-15, Deceth 4-6, Dodoxynol 5-12, Glycereth 7-26, Isoceteth 10-30, Isodeceth 4-6, Isolaureth 3-6, isosteareth 3-50, Laneth 5-75, Laureth 1-40, Nonoxynol 1-120, Nonylnonoxynol 5-150, Octoxynol 3-70, Oleth 2-50, PEG 4-350, Steareth 2-100, and Trideceth 2-10.

Specific examples of propoxylated alcohols are but are not limited to PPG-10 Cetyl Ether, PPG-20 Cetyl Ether, PPG-28 Cetyl Ether, PPG-30 Cetyl Ether, PPG-50 Cetyl Ether, PPG-2 Lanolin Alcohol Ether, PPG-5 Lanolin Alcohol Ether, PPG-10 Lanolin Alcohol Ether, PPG-20 Lanolin Alcohol Ether, PPG-30 Lanolin Alcohol Ether, PPG-4 Lauryl Ether, PPG-7 Lauryl Ether, PPG-10 Oleyl Ether, PPG-20 Oleyl Ether, PPG-23 Oleyl Ether, PPG-30 Oleyl Ether, PPG-37 Oleyl Ether, PPG-50 Oleyl Ether, PPG-11 Stearyl Ether, PPG-15 Stearyl Ether, PPG-2 Lanolin Ether, PPG-5 Lanolin Ether, PPG-10 Lanolin Ether, PPG-20 Lanolin Ether, PPG-30 Lanolin Ether, and PPG-1 Myristyl Ether.

Specific examples of ethoxylated/propoxylated alcohols are but are not limited to PPG-1 Beheneth-15, PPG-12 Capryleth-18, PPG-2-Ceteareth-9, PPG-4-Ceteareth-12, PPG-10-Ceteareth-20, PPG-1-Ceteth-1, PPG-1-Ceteth-5, PPG-1-Ceteth-10, PPG-1-Ceteth-20, PPG-2-Ceteth-1, PPG-2-Ceteth-5, PPG-2-Ceteth-10, PPG-2-Ceteth-20, PPG-4-Ceteth-1, PPG-4-Ceteth-5, PPG-4-Ceteth-10, PPG-4-Ceteth-20, PPG-5-Ceteth-20, PPG-8-Ceteth-1, PPG-8-Ceteth-2, PPG-8-Ceteth-5, PPG-8-Ceteth-10, PPG-8-Ceteth-20, PPG-2 C12-13 Pareth-8, PPG-2 C12-15 Pareth-6, PPG-4 C13-15 Pareth-15, PPG-5 C9-15 Pareth-6, PPG-6 C9-11 Pareth-5, PPG-6 C12-15 Pareth-12, PPG-6 C12-18 Pareth-11, PPG-3 C12-14 Sec-Pareth-7, PPG-4 C12-14 Sec-Pareth-5, PPG-5 C12-14 Sec-Pareth-7, PPG-5 C12-14 Sec-Pareth-9, PPG-1-Deceth-6, PPG-2-Deceth-3, PPG-2-Deceth-5, PPG-2-Deceth-7, PPG-2-Deceth-10, PPG-2-Deceth-12, PPG-2-Deceth-15, PPG-2-Deceth-20, PPG-2-Deceth-30, PPG-2-Deceth-40, PPG-2-Deceth-50, PPG-2-Deceth-60, PPG-4-Deceth-4, PPG-4-Deceth-6, PPG-6-Deceth-4, PPG-6-Deceth-9, PPG-8-Deceth-6, PPG-14-Deceth-6, PPG-6-Decyltetradeceth-12, PPG-6-Decyltetradeceth-20, PPG-6-Decyltetradeceth-30, PPG-13-Decyltetradeceth-24, PPG-20-Decyltetradeceth-10, PPG-2-Isodeceth-4, PPG-2-Isodeceth-6, PPG-2-Isodeceth-8, PPG-2-Isodeceth-9, PPG-2-Isodeceth-10, PPG-2-Isodeceth-12, PPG-2-Isodeceth-18, PPG-2-Isodeceth-25, PPG-4-Isodeceth-10, PPG-12-Laneth-50, PPG-2-Laureth-5, PPG-2-Laureth-8, PPG-2-Laureth-12, PPG-3-Laureth-8, PPG-3-Laureth-9, PPG-3-Laureth-10, PPG-3-Laureth-12, PPG-4 Laureth-2, PPG-4 Laureth-5, PPG-4 Laureth-7, PPG-4-Laureth-15, PPG-5-Laureth-5, PPG-6-Laureth-3, PPG-25-Laureth-25, PPG-7 Lauryl Ether, PPG-3-Myreth-3, PPG-3-Myreth-11, PPG-20-PEG-20 Hydrogenated Lanolin, PPG-2-PEG-11 Hydrogenated Lauryl Alcohol Ether, PPG-12-PEG-50 Lanolin, PPG-12-PEG-65 Lanolin Oil, PPG-40-PEG-60 Lanolin Oil, PPG-1-PEG-9 Lauryl Glycol Ether, PPG-3-PEG-6 Oleyl Ether, PPG-23-Steareth-34, PPG-30 Steareth-4, PPG-34-Steareth-3, PPG-38 Steareth-6, PPG-1 Trideceth-6, PPG-4 Trideceth-6, and PPG-6 Trideceth-8.

Alkoxylated fatty acids are formed when a fatty acid is reacted with an alkylene oxide or with a pre-formed polymeric ether. The resulting product may be a monoester, diester, or mixture thereof. Suitable ethoxylated fatty acid ester emollients suitable for use in the present invention are products of the addition of ethylene oxide to fatty acids. The product is a polyethylene oxide ester of a fatty acid. In one aspect of the invention, the ethoxylated fatty acid esters can be represented by the formula R-C(O)O(CH₂CH₂O)ₙ-H, wherein R represents the aliphatic residue of a fatty acid and n represents the number of molecules of ethylene oxide. In another aspect, n is an integer ranging from 2 to 50, 3 to 25 in another aspect, and 3 to 10 in a further aspect. In still another aspect of the invention, R is derived from a fatty acid containing 8 to 24 carbon atoms. In a still further aspect, R is derived from a fatty acid emollient set forth above. It is to be recognized that propoxylated and ethoxylated/propoxylated products of the foregoing fatty acids are also contemplated within the scope of the invention. Exemplary alkoxylated fatty acid esters include but are not limited to capric acid ethoxylate, lauric acid ethoxylate, myristic acid ethoxylate, stearic acid ethoxylate, oleic acid ethoxylate, coconut fatty acid ethoxylate, and polyethylene glycol 400 propoxylated monolaurate, wherein the number of ethylene oxide units in each of the foregoing ethoxylates can range from 2 and above in one aspect, and from 2 to 50 in another aspect. More specific examples of ethoxylated fatty acids are PEG-8 distearate (the 8 meaning the number of repeating ethylene oxide units), PEG-8 behenate, PEG-8 caprate, PEG-8 caprylate, PEG-8 caprylate/caprate, PEG cocoates (PEG without a number designation meaning that the number of ethylene oxide units ranges from 2 to 50), PEG-15 dicocoate, PEG-2 diisononanoate, PEG-8 diisostearate, PEG-dilaurates, PEG-dioleates PEG-distearates, PEG Ditallates, PEG-isostearates, PEG-jojoba acids, PEG-laurates, PEG-linolenates, PEG-myristates, PEG-oleates, PEG-palmitates, PEG-ricinoleates, PEG-stearates, PEG-tallates, and the like.

Guerbet ester emollients are formed from the esterification reaction of a Guerbet alcohol with a carboxylic acid. Guerbet ester emollients are commercially available from the Noveon Consumer Specialties Division of Lubrizol Advanced Materials, Inc. under product designations G-20, G-36, G-38, and G-66.

Lanolin and lanolin derivatives are selected from lanolin, lanolin wax, lanolin oil, lanolin alcohols, lanolin fatty acids, alkoxylated lanolin, isopropyl lanolate, acetylated lanolin alcohols, and combinations thereof. Lanolin and lanolin derivatives are commercially available from the Noveon Consumer Specialties Division of Lubrizol Advanced Materials, Inc. under the trade names Lanolin LP 108 USP, Lanolin USP AAA, Acetulan^{™}, Ceralan^{™}, Lanocerin^{™}, Lanogel^{™} (product designations 21 and 41), Lanogene^{™}, Modulan^{™}, Ohlan^{™}, Solulan^{™} (product designations 16, 75, L-575, 98, and C-24), Vilvanolin™ (product designations C, CAB, L-101, and P).

Other oily materials that are useful in combination with the polymers of the present invention include, for example, acetylated lanolin alcohols; lanolin alcohol concentrates; esters of lanolin fatty acids such as the isopropyl esters of lanolin fatty acid; polyol fatty acids; ethoxylated alcohols, such as ethoxylate and castor oils; sterols; sterol esters; sterol ethoxylates; and like materials. Many of such esters and ethoxylates are also useful as nonionic surfactants.

The emollient(s) can be utilized in an amount ranging from 0.5 weight percent to 30 weight percent based on the total weight of the personal care composition. In another embodiment, 0.1 weight percent to 25 weight percent of one or more emollients can be utilized, and in still another embodiment 5 weight percent to 20 weight percent of one or more emollients can be utilized. While emollients are generally employed in personal care compositions, they can be employed in home care, health care and institutional care compositions in the same weight ratios as set forth for personal care compositions so long as they effect a desired physical attribute (e.g., humectant properties) in such compositions.

Numerous ingredients are known in the art as conditioning agents for hair or skin, and humectants, and in addition to those previously discussed, nonlimiting examples include PCA (DL-pyrrolidone carboxylic acid) and its salts, such as lysine PCA, aluminum PCA, copper PCA, chitosan PCA, and the like, allantoin; urea; hyaluronic acid and its salts; ceramides; sorbic acid and its salts; sugars and starches and derivatives thereof; lactamide MEA; and the like.

While overlapping weight ranges for the various components and ingredients that can be contained in the compositions of the invention have been expressed for selected embodiments and aspects of the invention, it should be readily apparent that the specific amount of each component in the disclosed personal care, home care, health care, and institutional care compositions will be selected from its disclosed range such that the amount of each component is adjusted such that the sum of all components in the composition will total 100 weight percent. The amounts employed will vary with the purpose and character of the desired product and can be readily determined by one skilled in the formulation arts and from the literature.

It is also to be recognized that the choice and amount of ingredients in personal care, home care, health care and institutional care compositions that include the surfactant-polymer blend compositions of the invention can vary depending on the intended product and its function, as is well known to those skilled in the formulation arts. An extensive listing of ingredients and their conventional functions and product categories have been disclosed and can be readily ascertained from the literature, some of which can serve more than one function and that one or more of the disclosed ingredients can be combined with the surfactant-polymer compositions to obtain a desired personal care, home care, health care and institutional care composition.

### Examples

The following examples further illustrate the preparation and use of embodiments. The following examples demonstrate the benefits of using the polymer of this invention as main thickener in a number of surfactant formulations containing a variety of mixtures of anionic to amphoteric surfactants. Examples 1, 2, 3, 4 and 6 are not according to the invention.

### Test Methods

### Viscosity Measurement

Brookfield rotating spindle method: The viscosity of the test composition is measured 24 hours after formulation and reported as mPa·s, employing a Brookfield rotating spindle viscometer, Model DV-II (Brookfield Engineering Laboratories, Inc.), at about 20 revolutions per minute (rpm), at about 25°C ± 1°C (hereafter referred to as viscosity). Appropriate spindle sizes are set forth in the examples.

### Foam Volume Measurement

Foam volume is measured using a SITA Foam Tester, Model R-2000 (manufactured by SITA Messtechnik GmbH), which is interfaced with a personal computer loaded with a proprietary SITA measurement and analysis software application. Standard manufacturer's methods are followed for operation of the equipment. A known volume of a sample solution to be evaluated is placed in a temperature controlled vessel equipped with a stirrer and equilibrated to a temperature of 40°C ± 1°C. Once the temperature of the sample equilibrates, a foam cycle is run where the stirrer agitates the solution at 1000 rpm for 15 seconds to generate a foam. The foam is then measured by an array of sensor needles which measure the height at 16 points across the surface of the generated foam. The output is given as average millimeters of foam height per measure. For each test sample 60 foam cycles are measured. The height data is converted to foam volume (ml) by the integrated SITA software application and plotted. The foam volume measured over time without agitation cycles (foam stability) can also be determined and plotted.

### Example 1

A surfactant-polymer blend formulation is made using amino acid surfactants and a hydrophobically modified polymer. The 3 formulations set forth in the table below are each formulated with the following amino acid surfactants: sodium cocoyl glutamate (Amisoft^{®} CS-22, Ajinomoto), sodium cocoyl alaninate (Amilite® ACS-12, Ajinomoto), and potassium cocoyl glycinate (Amilite^{®} GCK-11).

### Example 1 - Formulation (System I)

| **ComponentNo.** | **Ingredient Name (Percent Active)** | **Wt. %** | **Wt.%** | **Wt. %** |
|---|---|---|---|---|
| 1 | Amino Acid Surfactant¹ (30%) | 50.0 | 50.0 | 50.0 |
| 2 | Acrylates/Beheneth-25 Methacrylate Copolymer² (30%) | 4.0 | 6.0 | 8.0 |
| 3 | Sodium Hydroxide (18% wt./wt. in D.I. water) | q.s. to pH 7.7 | q.s. to pH 7.7 | q.s. to pH 7.7 |
| 4 | Glydant Plus^{®} Preservative³ | 0.4 | 0.4 | 0.4 |
| 5 | Deionized Water | q.s. to 100 | q.s. to 100 | q.s. to 100 |
| ¹sodium cocoyl glutamate (Amisoft^{®} CS-22, Ajinomoto), sodium cocoyl alaninate (Amilite^{®} ACS-12, Ajinomoto), and potassium cocoyl glycinate (Amilite^{®} GCK-11). | | | | |
| ²INCI name for polymer component (b) of the present invention. The trade name is Novethix^{™} L-10 Polymer (Lubrizol Advanced Materials, Inc., Cleveland OH, USA). | | | | |
| ³INCI designation: DMDM Hydantoin (and) Iodopropynyl Butylcarbamate | | | | |

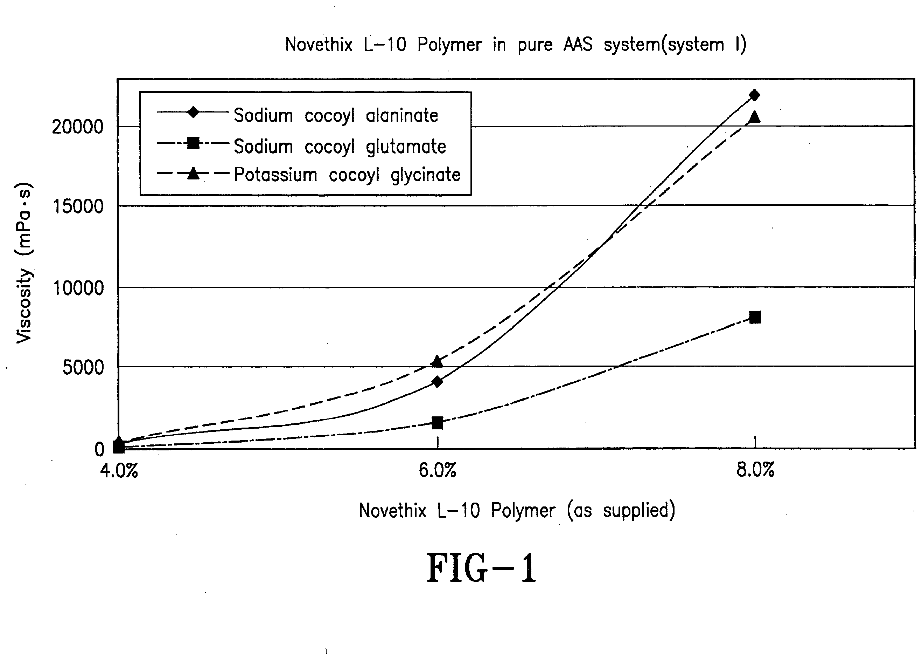

The Brookfield viscosity is measured (spindle #6) for each formulation and plotted. As shown in Figure 1, the hydrophobically modified polymer of the invention confers a high viscosity profile when used alone in aminoacid surfactant systems. Sodium cocoyl alaninate and potassium cocoyl glycinate systems have similar viscosity profiles. Sodium cocoyl glutamate, which is the most difficult to thicken, exhibits a lower viscosity profile.

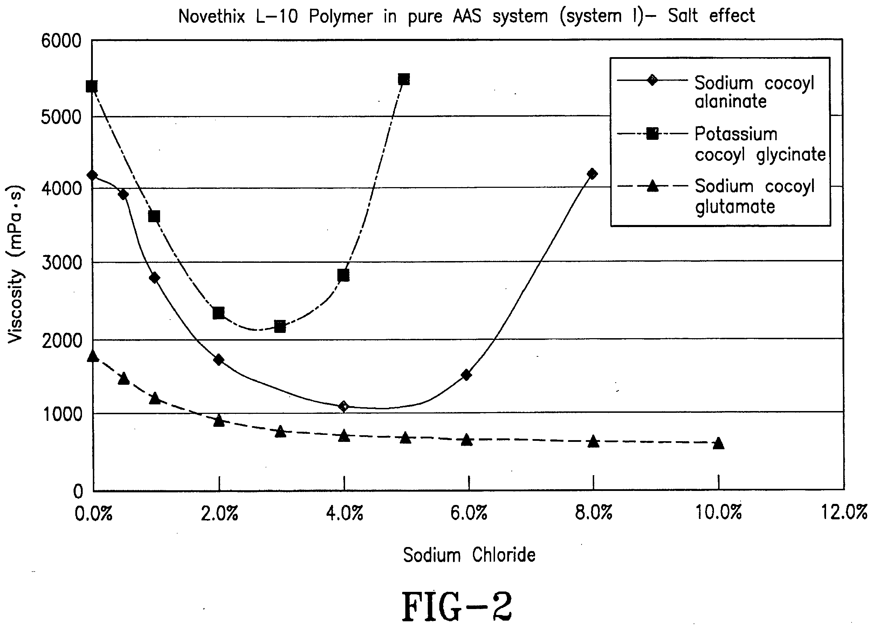

The 3 amino acid surfactant compositions containing sodium cocoyl glutamate, sodium cocoyl alaninate, and potassium cocoyl glycinate are each formulated with 6 weight percent of the Acrylates/Beheneth-25 Methacrylate Copolymer containing the components described in the table immediately above. A viscosity increasing electrolyte, sodium chloride, is added to the compositions. At typical electrolyte use levels (>0 to about 2.0 wt. %), a negative viscosity effect is observed as shown in Figure 2.

### Example 2

A surfactant-polymer blend formulation is made using amino acid surfactants and a hydrophobically modified polymer. An optional ethoxylated anionic surfactant (sodium laureth sulfate) and an optional (amphoteric surfactant cocamidopropyl) betaine is included in the formulation. As in Example 1, the 3 formulations set forth in the table below are each formulated with the following amino acid surfactants: sodium cocoyl glutamate (Amisoft^{®} CS-22, Ajinomoto), sodium cocoyl alaninate (Amilite^{®} ACS-12, Ajinomoto), and potassium cocoyl glycinate (Amilite^{®} GCK-11).

### Example 2 - Formulation (System II)

| **Component No.** | **Ingredient Name (Percent Active)** | **Wt.%** | **Wt.%** | **Wt.%** |
|---|---|---|---|---|
| 1 | Amino Acid Surfactant¹ (30%) | 33.3 | 33.3 | 33.3 |
| 2 | Sodium Laureth Sulfate (25%) (2 moles of ethoxylation) | 32.0 | 32.0 | 32.0 |
| 3 | Cocamidopropyl Betaine (38%) | 8.0 | 8.0 | 8.0 |
| 4 | Acrylates/Beheneth-25 Methacrylate Copolymer² (30%) | 4.0 | 6.0 | 8.0 |
| 5 | Sodium Hydroxide (18% wt./wt. in D.I. water) | q.s. to pH 7.7 | q.s. to pH 7.7 | q.s. to pH 7.7 |
| 6 | Glydant Plus^{®} Preservative³ | 0.4 | 0.4 | 0.4 |
| 7 | Deionized Water | q.s. to 100 | q.s. to 100 | q.s. to 100 |
| ^{1,2,3}As described in Example 1 | | | | |

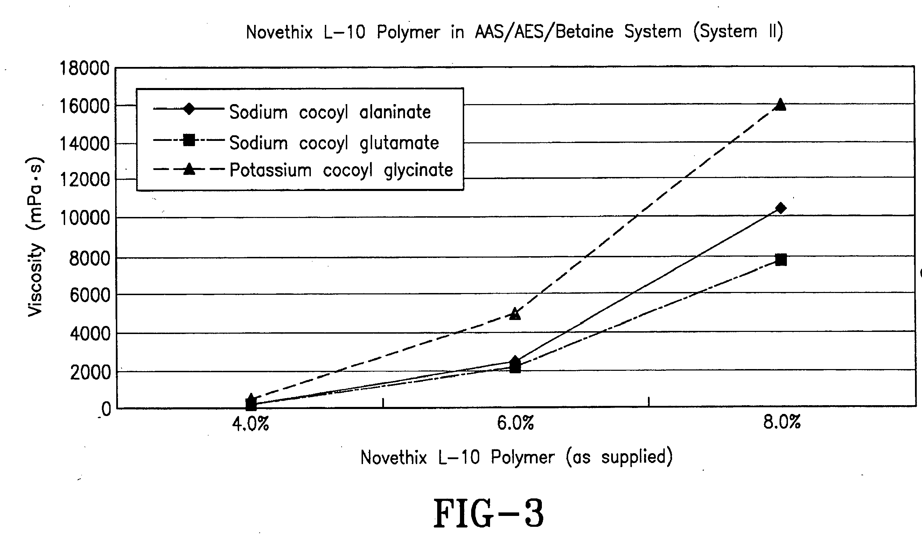

The Brookfield viscosity is measured (spindle #6) for each formulation and plotted. As shown in Figure 3, the hydrophobically modified polymer of the invention confers a high viscosity profile when used with a amino acid surfactant in combination with an ethoxylated alkyl sulfate and an amphoteric surfactant.

### Example 3

A surfactant-polymer blend formulation is made using amino acid surfactants and a hydrophobically modified polymer. An optional ethoxylated anionic surfactant (sodium lauroamphoacetate) and an optional amphoteric surfactant (cocamidopropyl betaine) is included in the formulation. As in Example 1, the 3 formulations set forth in the table below are individually formulated with the following amino acid surfactants: sodium cocoyl glutamate (Amisoft^{®} CS-22, Ajinomoto), sodium cocoyl alaninate (Amilite^{®} ACS-12, Ajinomoto), and potassium cocoyl glycinate (Amilite^{®} GCK-11).

### Example 3 - Formulation (System IIIA)

| **Component No.** | **Ingredient Name (Percent Active)** | **Wt. %** | **Wt. %** | **Wt. %** |
|---|---|---|---|---|
| 1 | Amino Acid Surfactant¹ (30%) | 33.3 | 33.3 | 33.3 |
| 2 | Sodium Lauroamphoacetate (25%) | 32.0 | 32.0 | 32.0 |
| 3 | Cocamidopropyl Betaine (38%) | 8.0 | 8.0 | 8.0 |
| 4 | Acrylates/Beheneth-25 Methacrylate Copolymer² (30%) | 4.0 | 6.0 | 8.0 |
| 5 | Glydant Plus^{®} Preservative³ | 0.4 | 0.4 | 0.4 |
| 6 | Deionized Water | q.s. to 100 | q.s. to 100 | q.s. to 100 |
| ^{1,2,3}As described in Example 1 | | | | |

### Example 4:

A surfactant-polymer blend formulation is made using amino acid surfactants and a hydrophobically modified polymer. An optional nonionic surfactant (decyl glucoside) and an optional amphoteric surfactant (cocamidopropyl betaine) is included in the formulation. As in Example 1, the 3 formulations set forth in the table below are individually formulated with the following amino acid surfactants: sodium cocoyl glutamate (Amisoft® CS-22, Ajinomoto), sodium cocoyl alaninate (Amilite® ACS-12, Ajinomoto), and potassium cocoyl glycinate (Amilite® GCK-11).

### Example 4 - Formulation (System IIIB)

| **Component No.** | **Ingredient Name (Percent Active)** | **Wt. %** | **Wt. %** | **Wt. %** |
|---|---|---|---|---|
| 1 | Amino Acid Surfactant¹ (30%) | 33.3 | 33.3 | 33.3 |
| 2 | Decyl Glucoside (53%) | 32.0 | 32.0 | 32.0 |
| 3 | Cocamidopropyl Betaine (38%) | 8.0 | 8.0 | 8.0 |
| 4 | Acrylates/Beheneth-25 Methacrylate Copolymer² (30%) | 4.0 | 6.0 | 8.0 |
| 5 | Sodium Hydroxide (18% wt./wt. in D.I. water) | q.s. to pH 7.0 | q.s. to pH 7.0 | q.s. to pH 7.0 |
| 6 | Glydant Plus® Preservative³ | 0.4 | 0.4 | 0.4 |
| 7 | Deionized Water | q.s. to 100 | q.s. to 100 | q.s. to 100 |
| ^{1,2,3}As described in Example 1 | | | | |

### Example 5:

Potassium cocoyl glycinate is formulated as set forth in Example 1 containing 4, 6, and 8 weight percent of a hydrophobically modified polymer of the invention. The pH of the formulation is adjusted with sodium hydroxide (18% wt./wt. in D.I. water) to pH 7.7. To each formulation, 2.0 weight percent (100 percent active material) of an esterified alkoxylated methyl glucoside (PEG-120 Methyl Glucose Dioleate) is added. A second set of comparative test samples is identically formulated except that the hydrophobically modified polymer is not added and the amount of esterified alkoxylated methyl glucoside is increased to 3.0 weight percent (100 percent active material) of the total formulation.

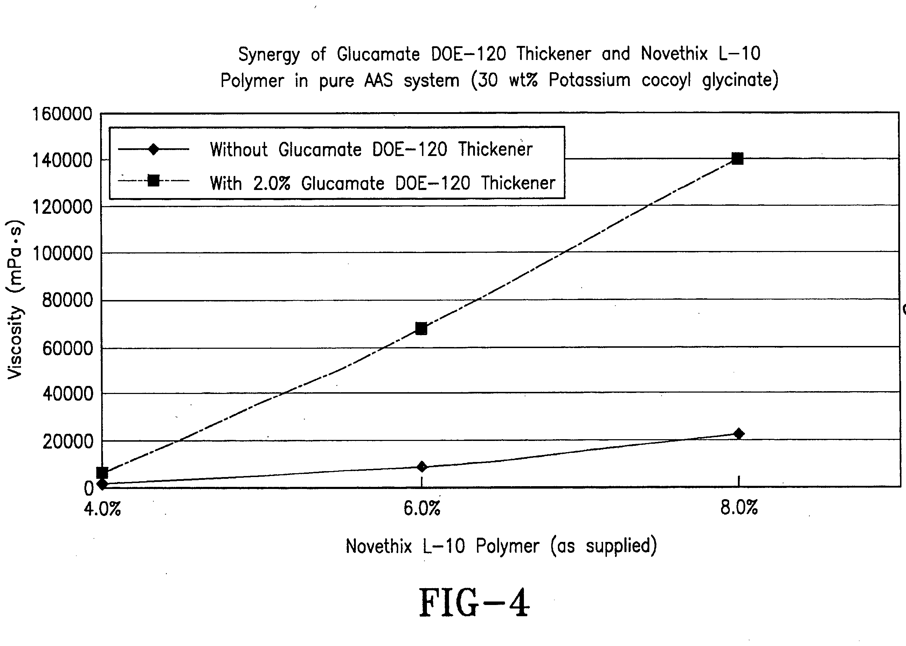

The 24 hour Brookfield viscosity of each formulation is measured and plotted as shown in Figure 4. In amino acid surfactant formulations containing 3.0 weight percent of the esterified alkoxylated methyl glucoside the viscosity increase was minimal. However, in amino acid surfactant formulations containing a hydrophobically modified polymer of the invention and only 2.0 weight percent of the esterified alkoxylated methyl glucoside, the viscosity profiles of the amino acid surfactant systems are significantly increased, demonstrating a synergistic viscosity increasing effect.

### Example 6

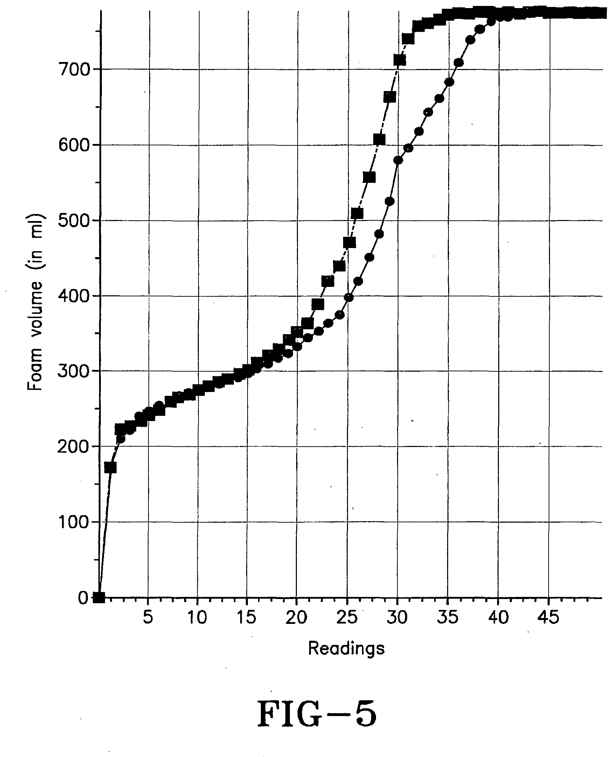

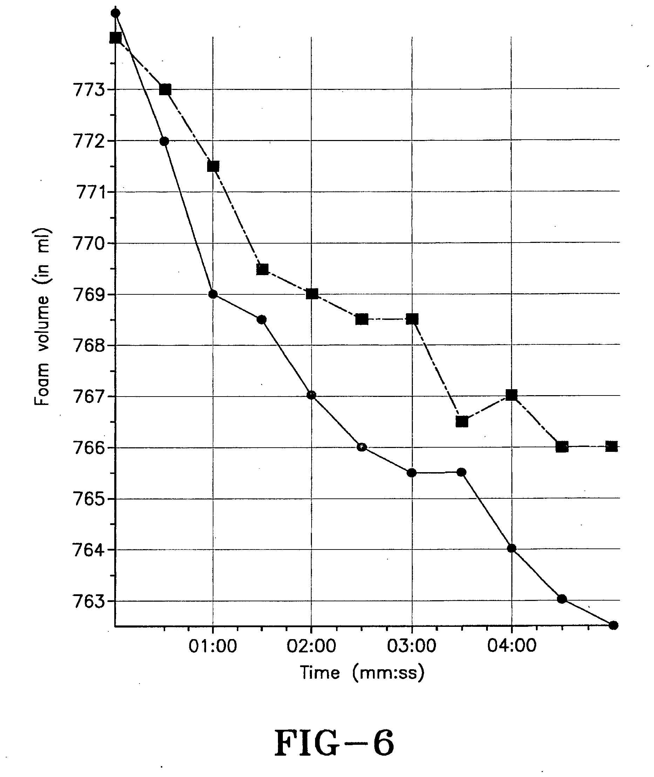

The formulation of Example 3 containing the sodium cocoyl glycinate surfactant and 8.0 weight percent of Acrylates/Beheneth-25 Methacrylate Copolymer (as supplied) is tested for foaming properties using the foam volume measurement protocol set forth above. The pH of the formulation is adjusted with sodium hydroxide (18% wt./wt. in D.I. water) to pH 7.0. A comparative formulation blank (containing no Acrylates/Beheneth-25 Methacrylate Copolymer) is also tested. Foam volume measurements and foam stability plots are set forth in Figures 5 and 6, respectively. In both figures foam volume in ml (y axis) is plotted vs. time in min. (x axis). The points represented by squares correspond to formulation of the invention (Example 3) and points represented by ovals correspond to the comparative blank formulation.

## Claims

1. A surfactant-polymer blend comprising:
(a) at least one amino acid surfactant selected from the mono-and di-carboxylate salts of N-acylated glutamic acid; the carboxylate salts of N-acylated alanine; the carboxylate salts of N-acylated glycine; the carboxylate salts of N-acylated sarcosine; and mixtures thereof; and
(b) at least one hydrophobically-modified polymer polymerized from a monomer mixture comprising:
(i) from 30 weight percent to 50 weight percent of at least one α,β-ethylenically unsaturated carboxylic acid monomer;
(ii) from 30 weight percent to 70 weight percent of at least one nonionic, co-polymerizable α,β-ethylenically unsaturated monomer selected from C₁-C₄ alkyl acrylates and methacrylates; and
(iii) from 7.5 weight percent to 15 weight percent of at least one ethylenically unsaturated hydrophobically-modified associative monomer; and
(c) an esterified alkoxylated methyl glucoside.

2. The surfactant-polymer blend of claim 1, wherein the blend further comprises one or more nonionic surfactants, one or more cationic surfactants, one or more amide surfactants, or a mixture thereof.

3. The surfactant-polymer blend of claim 1 to 2, wherein said at least one hydrophobically-modified polymer is polymerized from a monomer mixture comprising:
(i) at least one α,β-ethylenically unsaturated carboxylic acid monomer selected from acrylic acid, methacrylic acid, and mixtures thereof;
(ii) at least one nonionic, co-polymerizable α,β-ethylenically unsaturated monomer selected from C₁ to C₄ alkyl acrylate, C₁ to C₄ alkyl methacrylate, and mixtures thereof; and
(iii) at least one ethylenically unsaturated hydrophobically-modified associative monomer selected from cetyl polyethoxylated (meth)acrylate, cetearyl polyethoxylated (meth)acrylate, stearyl polyethoxylated (meth)acrylate, arachidyl polyethoxylated (meth)acrylate, behenyl polyethoxylated methacrylate, cerotyl polyethoxylated (meth)acrylate, montanyl polyethoxylated (meth)acrylate, melissyl polyethoxylated (meth)acrylate, lacceryl polyethoxylated (meth)acrylate, nonylphenol polyethoxylated (meth)acrylate, tristyrylphenol polyethoxylated (meth)acrylate, palmyl polyethoxylated itaconate, stearyl polyethoxylated itaconate, behenyl polyethoxylated itaconate, palmyl polyethoxylated maleate, stearyl polyethoxylated maleate, behenyl polyethoxylated maleate, palmyl polyethoxylated allyl ether, stearyl polyethoxylated allyl ether, behenyl polyethoxylated allyl ether, wherein the polyethoxylated portion of the foregoing monomers contain from 15 to 60 ethylene oxide repeating units.

4. The surfactant-polymer blend of any of claims 1 to 3, wherein said at least one hydrophobically-modified polymer is Acrylates/Beheneth-25 Methacrylate Copolymer.

5. The surfactant-polymer blend of claim 1, wherein said at least one amino acid surfactant salt is selected from the sodium, potassium, ammonium and TEA salt thereof.

6. The surfactant-polymer blend of any of claims 1 to 5, wherein said at least one amino acid surfactant is selected from sodium cocoyl glutamate, sodium cocoyl alaninate, potassium cocoyl glycinate, and mixtures thereof.

## Patentansprüche

1. Tensid-Polymer-Gemisch, umfassend:
(a) wenigstens ein Aminosäure-Tensid, das aus den Mono- und Dicarboxylatsalzen von N-acylierter Glutaminsäure, den Carboxylatsalzen von N-acyliertem Alanin, den Carboxylatsalzen von N-acyliertem Glycin, den Carboxylatsalzen von N-acyliertem Sarcosin und Gemischen davon ausgewählt ist; und
(b) wenigstens ein hydrophob modifiziertes Polymer, das aus einem Monomergemisch polymerisiert ist, welches Folgendes umfasst:
(i) 30 Gew.-% bis 50 Gew.-% wenigstens eines α,β-ethylenisch ungesättigten Carbonsäure-Monomers;
(ii) 30 Gew.-% bis 70 Gew.-% wenigstens eines nichtionischen copolymerisierbaren α,β-ethylenisch ungesättigten Monomers; und
(iii) 7,5 Gew.-% bis 15 Gew.-% wenigstens eines ethylenisch ungesättigten hydrophob modifizierten assoziativen Monomers das ausgewählt ist aus C₁- bis C₄-Alkylacrylaten und Methacrylaten; und
(c) ein verestertes alkoxyliertes Methylglucosid.

2. Tensid-Polymer-Gemisch gemäß Anspruch 1, wobei das Gemisch weiterhin ein oder mehrere nichtionische Tenside, ein oder mehrere kationische Tenside, ein oder mehrere Amid-Tenside oder ein Gemisch davon umfasst.

3. Tensid-Polymer-Gemisch gemäß Anspruch 1 oder 2, wobei das wenigstens eine hydrophob modifizierte Polymer aus einem Monomergemisch polymerisiert ist, welches Folgendes umfasst:
(i) wenigstens ein α,β-ethylenisch ungesättigtes Carbonsäure-Monomer, das aus Acrylsäure, Methacrylsäure und Gemischen davon ausgewählt ist;
(ii) wenigstens ein nichtionisches copolymerisierbares α,β-ethylenisch ungesättigtes Monomer, das aus C₁- bis C₄-Alkylacrylat, C₁- bis C₄-Alkylmethacrylat und Gemischen davon ausgewählt ist; und
(iii) wenigstens ein ethylenisch ungesättigtes hydrophob modifiziertes assoziatives Monomer, das aus polyethoxyliertem Cetyl(meth)-acrylat, polyethoxyliertem Cetearyl(meth)acrylat, polyethoxyliertem Stearyl(meth)acrylat, polyethoxyliertem Arachidyl(meth)acrylat, polyethoxyliertem Behenylmethacrylat, polyethoxyliertem Cerotyl-(meth)acrylat, polyethoxyliertem Montanyl(meth)acrylat, polyethoxyliertem Melissyl(meth)acrylat, polyethoxyliertem Lacceryl-(meth)acrylat, polyethoxyliertem Nonylphenol(meth)acrylat, polyethoxyliertem Tristyrylphenol(meth)acrylat, polyethoxyliertem Palmylitaconat, polyethoxyliertem Stearylitaconat, polyethoxyliertem Behenylitaconat, polyethoxyliertem Palmylmaleat, polyethoxyliertem Stearylmaleat, polyethoxyliertem Behenylmaleat, polyethoxyliertem Palmylallylether, polyethoxyliertem Stearylallylether, polyethoxyliertem Behenylallylether ausgewählt ist, wobei der polyethoxylierte Teil der obigen Monomere 15 bis 60 Ethylenoxid-Repetiereinheiten enthält.

4. Tensid-Polymer-Gemisch gemäß einem der Ansprüche 1 bis 3, wobei es sich bei dem wenigstens einen hydrophob modifizierten Polymer um Acrylate/Beheneth-25-methacrylat-Copolymer handelt.

5. Tensid-Polymer-Gemisch gemäß Anspruch 1, wobei das wenigstens eine Aminosäure-Tensidsalz aus dem Natrium-, Kalium-, Ammonium- und TEA-Salz davon ausgewählt ist.

6. Tensid-Polymer-Gemisch gemäß einem der Ansprüche 1 bis 5, wobei das wenigstens eine Aminosäure-Tensid aus Natriumcocoylglutamat, Natriumcocoylalaninat, Kaliumcocoylglycinat und Gemischen davon ausgewählt ist.

## Revendications

1. Mélange de tensioactif-polymère comprenant :
(a) au moins un tensioactif acide aminé choisi parmi les sels mono- et di-carboxylates d'acide glutamique N-acylé ; les sels carboxylates d'alanine N-acylée ; les sels carboxylates de glycine N-acylée ; les sels carboxylates de sarcosine N-acylée ; et leurs mélanges ; et
(b) au moins un polymère à modification hydrophobe polymérisé à partir d'un mélange de monomères comprenant :
(i) de 30 % en poids à 50 % en poids d'au moins un monomère d'acide carboxylique à insaturation α,β-éthylénique ;
(ii) de 30 % en poids à 70 % en poids d'au moins un monomère à insaturation α,β-éthylénique non ionique copolymérisable choisi parmi les acrylates et les méthacrylates d'alkyle en C₁ à C₄ ; et
(iii) de 7,5 % en poids à 15 % en poids d'au moins un monomère associatif à modification hydrophobe à insaturation éthylénique ; et
(c) un méthylglucoside alcoxylé estérifié.

2. Mélange de tensioactif-polymère selon la revendication 1, lequel le mélange comprend en outre un ou plusieurs tensioactifs non ioniques, un ou plusieurs tensioactifs cationiques, un ou plusieurs tensioactifs amides, ou un mélange de ceux-ci.

3. Mélange de tensioactif-polymère selon la revendication 1 ou 2, dans lequel ledit au moins un polymère à modification hydrophobe est polymérisé à partir d'un mélange de monomères comprenant :
(i) au moins un monomère d'acide carboxylique à insaturation α,β-éthylénique choisi parmi l'acide acrylique, l'acide méthacrylique, et leurs mélanges ;
(ii) au moins un monomère à insaturation α,β-éthylénique non ionique, copolymérisable, choisi parmi un acrylate d'alkyle en C₁ à C₄, un méthacrylate d'alkyle en C₁ à C₄, et leurs mélanges ; et
(iii) au moins un monomère associatif à modification hydrophobe à insaturation éthylénique choisi parmi le (méth)acrylate de cétyle polyéthoxylé, le (méth)acrylate de cétéaryle polyéthoxylé, le (méth)acrylate de stéaryle polyéthoxylé, le (méth)acrylate d'arachidyle polyéthoxylé, le méthacrylate de béhényle polyéthoxylé, le (méth)acrylate de cérotyle polyéthoxylé, le (méth)acrylate de montanyle polyéthoxylé, le (méth)acrylate de mélissyle polyéthoxylé, le (méth)acrylate de laccéryle polyéthoxylé, le (méth)acrylate de nonylphénol polyéthoxylé, le (méth)acrylate de tristyrylphénol polyéthoxylé, l'itaconate de palmyle polyéthoxylé, l'itaconate de stéaryle polyéthoxylé, l'itaconate de béhényle polyéthoxylé, le maléate de palmyle polyéthoxylé, le maléate de stéaryle polyéthoxylé, le maléate de béhényle polyéthoxylé, l'éther allylique de palmyle polyéthoxylé, l'éther allylique de stéaryle polyéthoxylé, l'éther allylique de béhényle polyéthoxylé, la partie polyéthoxylée des monomères ci-dessus contenant de 15 à 60 motifs répétitifs oxyde d'éthylène.

4. Mélange de tensioactif-polymère selon l'une quelconque des revendications 1 à 3, dans lequel ledit au moins un polymère à modification hydrophobe est un copolymère d'acrylate/méthacrylate de béhénéth-25.

5. Mélange de tensioactif-polymère selon la revendication 1, dans lequel ledit au moins un sel de tensioactif acide aminé est choisi parmi les sels de sodium, de potassium, d'ammonium et de TEA de celui-ci.

6. Mélange de tensioactif-polymère selon l'une quelconque des revendications 1 à 5, dans lequel ledit au moins un tensioactif acide aminé est choisi parmi le cocoylglutamate de sodium, le cocoylalaninate de sodium, le cocoylglycinate de potassium, et leurs mélanges.
